# EUROPEAN PATENT APPLICATION

(11) **EP 2 281 557 A2**
(43) Date of publication of application: **09.02.2011**
(21) Application number: 09738963.9
(22) Date of filing: 28.04.2009
(51) Int. Cl.: A61K 9/22, A61K 9/00, A61K 9/20, A61K 9/48

(54) **PHARMACEUTICAL FORMULATION CONTAINING ANGIOTENSIN-II RECEPTOR BLOCKER**

(30) Priority: 29.04.2008 KR 20080040002
(71) Applicant: HanAll Biopharma Co., Ltd., Daejeon 306-120 (KR)
(72) Inventor: KIM, Sung Wuk, Seongnam-si Gyeonggi-do 463-802 (KR); JUN, Sung Soo, Seongnam-si Gyeonggi-do 463-777 (KR); JO, Young Gwan, Daejeon 305-811 (KR); KOO, Ja Seong, Daejeon 305-756 (KR); LEE, Ah Ram, Incheon 403-765 (KR); SON, Jae Woon, Suwon-si Gyeonggi-do 442-753 (KR); KIM, Jeong Taek, Anyang-si Gyeonggi-do 430-838 (KR)
(74) Representative: Pohlman, Sandra M.
(86) International application number: PCT/KR2009/002225
(87) International publication number: WO 2009/134057

(57) **Abstract**

The present invention provides a pharmaceutical formulation containing an angiotensin-II receptor blocker and a release-control material as a pharmacologically active ingredient and a pharmaceutical formulation comprising an immediate-release compartment and an extended-release compartment. The immediate-release compartment contains an agent as a pharmacologically active ingredient for preventing and inhibiting hepatitis and the extended-release compartment has an angiotensin-II receptor blocker as a pharmacologically active ingredient. The formulation of the present invention maximizes the effectiveness on pharmacologically and clinically lowering blood pressure and preventing complications when taking the formulation, helps to avoid interaction with a drug which is metabolized by the same enzyme in the liver, and prevents and inhibits the incidence of drug-induced hepatitis which is caused by drug administration for a long time.

## Description

### TECHNICAL FIELD

The present invention relates to a pharmaceutical formulation which is capable of further enhancing drug efficacy when used as a single drug. Further, the present invention relates to a pharmaceutical formulation which is capable of reducing adverse side effects and further enhancing drug efficacy when concurrently administered with other drugs.

### BACKGROUND ART

Hypertension is a disorder which serves as a primary cause of death due to cardiovascular diseases and causes fatal diseases such as cerebral stroke, myocardial infarction, congestive heart failure, and peripheral vascular diseases. Blood pressure in a human is not constantly maintained day and night. This is because predictable changes occurring over 24 hours in the environment and physiological diversity create circadian biorhythmic patterns of blood pressure and heartbeat. The blood pressure of non-dippers whose blood pressure is not reduced in their sleep suddenly rises when getting up in the morning and reaches a peak during a time period where daytime activity is initiated.
In a renin-angiotensin-aldosterone system which is one of various mechanisms responsible for the elevation of blood pressure, angiotensinogen is converted into angiotensin I by the action of renin secreted in the kidney, and angiotensin I is converted into angiotensin II by the action of an angiotensin converting enzyme. The converted angiotensin II is involved in vasoconstriction and renal resorption of NaCl and water. The renin-angiotensin-aldosterone system works primarily in the night and actively produces aldosterone and angiotensin II which are vasoconstriction-inducing agents, which consequently contributes to the elevation of blood pressure from the night to the morning [The cardiovascular continuum and renin-angiotensin-aldosterone system blockade. J Hypertens Suppl. 2005 Apr; 23(1): S9-17].

Through the action of such a renin-angiotensin-aldosterone system, the mortality of hypertensive patients due to myocardial infarction, cerebrovascular diseases and heart failure reaches the highest level in the early morning time during which daily activities take place (from 06 a.m. to noon) [Morning blood pressure peak, QT intervals, and sympathetic activity in hypertensive patients. Hypertension 41 (2): 237-243].

The angiotensin-II-receptor blocker has been elucidated up to now as a drug exerting hypotensive effects on both systolic and diastolic phases of the myocardium, by blocking the binding of angiotensin-II, one of fundamental substances responsible for vasoconstriction, to an AT1 receptor out of angiotensin receptors. As the angiotensin-II-receptor blocker, there are about 10 kinds of compound groups that are frequently clinically applied, including pharmaceutically acceptable salts. Further, these compounds are used alone or in combination with an angiotensin converting enzyme inhibitor or the like exhibiting hypotensive effects through a similar mechanism, on mild to severe patients suffering from various symptoms associated with hypertension [Angiotensin 2 Receptor Antagonist: An Overview, Am J Health-Syst Pharm 57(13): 1231-1238, 2000].

Among these angiotensin-II-receptor blockers, losartan, valsartan, irbesartan, candesartan, telmisartan, eprosartan, olmesartan, and the like have been commercialized and have rapidly grown as anti-hypertension drugs over the past several years. In addition, effects of these drugs are also verified through various clinical trials [Pharmacologic, Pharmacokinetic, and Therapeutic Difference Among angiotensin-II-receptor Antagonist: Pharmacotherapy 20(2): 130-139, 2000].
As demonstrated through such various clinical trials, these angiotensin-II-receptor blockers are equivalent in hypotensive effects on myocardial systolic and diastolic phases at an optimum dose, even though they exhibit pharmacokinetic differences in *in vivo* metabolic pathways and half lives. Further, it is also known that these angiotensin-II-receptor blockers are similar in effects obtained by the same receptor blocker, such as prevention and treatment of secondary heart failure associated with various symptoms of hypertension, prevention and treatment of post-myocardial infarction arrhythmia and heart failure, prevention and treatment of diabetic complications, prevention and treatment of renal failure, prevention and treatment of cerebral stroke, anti-platelet effects, arteriosclerosis-preventive effects, suppression of harmful effects of aldosterone, reduction of effects of metabolic syndromes, and effects of preventing circulatory diseases from becoming worse in a chain-like manner.
With regard to the treatment of hypertension irrespective of the type thereof upon taking into consideration the nature of disease, maximum effects of medication should be exerted in the early morning during which daily blood pressure becomes highest, and more preferably evening administration of the drug is recommended so as to keep blood pressure-lowering effects for sleeping hours where the synthesis of renin which is a vasoconstriction-inducing fundamental material is performed and from the night to the early morning during which the synthesis of angiotensin and aldosterone directly responsible for the elevation of blood pressure reaches the peak [Patterns of blood pressure response: Day and night variations; Am J Hypertens. 2001 April; 14 (4, Part 2) 262A, Preventing increase in early morning blood pressure, heart rate, and the rate-pressure product with controlled onset extended release verapamil at bedtime versus enalapril, losartan, and placebo on rising; Am Heart J. 2002 October; 144 (4): 657-665]. The renin-angiotensin-aldosterone system, which is responsible for such elevation of blood pressure, works primarily during night to actively produce aldosterone and angiotensin II which are vasoconstriction-inducing agents, so an angiotensin-II-receptor blocker, which inhibits the synthesis of aldosterone and selectively blocks an AT1 receptor out of angiotensin receptors, needs to be administered at midnight.

However, conventional angiotensin-II-receptor blocker single drugs, which are immediately released *in vivo* after administration thereof, are generally administered in the morning or evening mealtime. Administration of the drug at the morning mealtime cannot provide effective inhibitory effects on the synthesis of vasoconstriction inducers aldosterone and angiotensin II which occurs during the sleeping period of the corresponding day. Further, administration of the drug at the evening mealtime cannot provide intensive hypotensive effects up to the early morning time period where daily blood pressure on the next day reaches its peak. Accordingly, with the use of an angiotensin-II-receptor blocker single drug exhibiting immediate *in vivo* release after administration thereof, it is impossible to achieve maximum hypotensive effects in the early morning time period where daily blood pressure reaches its peak. Further, upon night administration of the angiotensin-II-receptor blocker single drug exhibiting immediate *in vivo* release in order to administer the drug at a time where maximum hypotensive effects are exerted, the compliance of patients is lowered, thus making medication compliance difficult and consequently rendering treatment of the disease to be difficult.

To this end, there is a need for the development of a formulation which is capable of enhancing the patients' medication compliance.

The onset of hypertensive diseases is largely in aged people over the age of 40 and is frequently accompanied by other secondary diseases. Among drugs which are additionally administered for simultaneous treatment of various diseases as well as hypertensive diseases, there are drugs which are metabolized by hepatic cytochrome. Examples of such additional drugs include a lipid inhibitor such as simvastatin or atorvastatin; an antihypertensive agent such as amlodipine, felodipine, carvedilol or aliskiren; an antihistamine agent such as loratadine or azelastine; an analgesic agent such as acetaminophen or tramadol; an antidepressant such as amitriptyline or imipramine; an anticoagulant such as warfarin; an anti-emetic agent such as chlorpromazine or granisetron; an anticonvulsant such as carbamazepine; an anti-acne agent (retinoid) such as isotretinoin; an antipsychotic agent such as risperidone; an antidepressant such as propyne or venlafaxine; an HIV-antiviral agent (protease inhibitor) such as fosamprenavir; an antifungal agent such as itraconazole; a thiazolidinedione antidiabetic agent such as pioglitazone; an anti-obesity agent such as sibutramine; an anti-erectile dysfunction agent such as sildenafil; and an anti-incontinence agent such as tolterodine.

Methods for treating hypertension and accompanying diseases through the administration of an angiotensin-II-receptor blocker are already known (US Patent Application No. 2003-0158244 A1, and International Patent Publication Nos. WO 95/26188, WO 97/37688, WO 99/11260, WO 04/062729, WO 06/040085 and WO 2002/40007).

For example, an HMG-CoA reductase inhibitor simvastatin, which is one of drugs which are metabolized by hepatic cytochrome, among drugs which have been used in the treatment of hypertensive diseases as well as other diseases, and an angiotensin-II-receptor blocker losartan are described to have the following problems.

Losartan is absorbed and transported to the liver. Some of losartan is released into blood in the form of an active losartan molecule, reaching a peak blood concentration within 1 hour. However, the remaining portion of losartan is metabolized by two hepatic enzymes, cytochrome P450 2C9 and 3A4 and is therefore converted into losartan carboxylic acid having a higher activity, reaching the highest blood concentration after 3 to 4 hours. That is, the total pharmacological activity of losartan is the pharmacological action of a mixture of losartan and losartan carboxylic acid (losartan's active metabolite). The active metabolite losartan carboxylic acid exhibits a pharmacological activity 40 times higher than that of losartan. The plasma clearance rate is 600 mL/min for losartan and 50 mL/min for losartan carboxylic acid (active metabolite), suggesting that the active metabolite shows a slower clearance rate, and thus plays an important role in maintaining the long-lasting action time. Simvastatin, which is used in combination with losartan, is a statin-based lipid-reducing agent, which inhibits the conversion of 3-hydroxy-3-methylglutaryl-coenzyme A (HMG-CoA) into mevalonate by the action of HMG-CoA reductase, thus showing the effects of inhibiting the synthesis of cholesterol in the liver and reducing low-density lipoprotein cholesterol (LDL-C) levels [Lancet 1995; 346: 750-753, Am J Cardiol 1998; 82: 57T-59T, Am J Cardiol 1995; 76: 107C-112C, Hypertens Res 2003; 26: 699-704, Hypertens Res 2003; 26: 273-280.] Br Med Bull 2001; 59: 3-16, Am J Med 1998; 104 (Suppl 1): 6S-8S, Clin Pharmacokinet 2002; 41: 343-370]. Due to having such effects, simvastatin is known as a drug which is very effective in the treatment and prevention of composite hyperlipidemia, atherosclerosis and coronary heart diseases ["Scandinavian Simvastatin Survival Study" published in the Lancet, vol. 344, (1994), p. 1383-89; Lancet, 1994; 344: 1383-1389]. Further, simvastatin is inactive in itself, but converts into an active form β-hydroxyacid in the liver and is then metabolized by the hepatic enzyme cytochrome P450 3A4, works in the liver and is excreted from the liver. When the remaining simvastatin, which was not metabolized by cytochrome P450 3A4, is present at a high level in blood, this leads to a further increase in the risk of causing myopathies, such as myolysis, which is the adverse side effect of simvastatin [Drug Metab Dispos 1990; 18: 138-145, Drug Metab Dispos 1990; 18: 476-483, Drug Metab Dispos 1997; 25: 1191-1199, Clin Pharmacol Ther 1998; 63: 332-341 ; Clin Pharmacol Ther 1998; 64: 177-182 ; Physicians Desk Reference 2006 (Zocor) ; J Pharmacol Exp Ther 1997; 282: 294-300 ; Pharmacol Exp Ther 1999; 290: 1116-1125 ; Life Sci 2004; 76: 281-292].
As described above, when a drug metabolized by hepatic cytochrome should be additionally administered concurrently with an angiotensin-II-receptor blocker, the angiotensin-II-receptor blocker and the cytochrome-metabolized drug exhibit competitive action therebetween *in vivo,* so either of them is not sufficiently metabolized, thus causing the problem of drug interaction such as decreased drug efficacy or increased adverse side effects. As a consequence, treatment of both hypertensive diseases and other diseases by combination administration of drugs may be not obtained, and the problem of drug interaction may also occur upon concurrent administration of drugs.
Further, in order to address the problem of interactions between metabolic enzymes and achieve administration of drugs at the time period where maximum pharmacological effects of the angiotensin-II-receptor blocker are exerted, when the angiotensin-II-receptor blocker exhibiting immediate *in vivo* release is administered 2 to 4 hours after medication of a drug metabolized by hepatic cytochrome, the compliance of patients is reduced, thus making it difficult to treat the disease of interest.

In particular, there are large numbers of drugs inhibiting cytochrome P450 enzymes that metabolize and activate losartan. For instance, co-administration of a cytochrome P450 3A4-inhibiting drug (such as indinavir, nelfinavir, ritonavir, clarithromycin, itraconazole, ketoconazole, nefazodone, saquinavirm telithromycin, aprepitan, erythromycin, fluconazole, verapamil, cimetidine, amiodaronem chloramphenicol, delaviridinem diethyldithiocarbamate, fluvoxamine, gestodene, imatinib, mibefradil, mifepristone, norfloxacin, norflouxetine, or voriconazole) or a CYP2C9-inhibiting drug (such as fluconazole, amiodarone, fenofibratem fluvastatin, fluvoxamine, isoniazid, phenylbutazone, probenecid, sertraline, sulfamethoxazole, sulfaphenazole, teniposide, or voriconazole) with an angiotensin-II-receptor blocker losartan leads to inhibition of the losartan activity and consequently desired pharmacological effects of losartan cannot be sufficiently exerted.

To this end, there is a need for the development of a formulation which can be freely co-administered with these drugs.

Further, it is reported that the majority of drugs undergoing hepatic metabolism may cause inflammation due to destruction of the liver as adverse side effects thereof. In particular, since the treatment of hypertension is focused on exertion of drug-induced hypotensive effects and inhibition of hypertension-induced complications, not focusing on the treatment of underlying pathological causes, this requires long-term medication and co-administration with other drugs. Since drug-induced hepatitis is dose-dependent, administration of several kinds of drugs serves to repeatedly destroy the liver. Further, even though it is administered alone, long-term medication of an angiotensin-II-receptor blocker increases the risk of hepatitis. Hepatitis is the destruction of hepatic parenchymal cells and lobules. This is referred to as oxidative stress, and drug-induced free radicals generated in the liver lead to oxidation of lipids to lipid peroxides and the lipid peroxides in turn lead to generation of free radicals in geometric progression, thus resulting in hepatic destruction. Even though hepatic destruction is prevented if a sufficient amount of an antioxidant is present in the liver, hypertensive patients are usually deficient in antioxidants throughout all the tissues.

The initial pathological symptom caused by oxidative stress is fatty liver where more than 5% of the hepatocyte weight turns into lipid peroxides or the like. The fatty liver quickly degrades into liver-destroying hepatitis, thus resulting in necrosis of hepatocytes. Upon necrosis of hepatocytes, enzymes such as GPT and GOT, playing a role in hepatocytes, are liberated and discharged into blood. Therefore, the state of hepatitis is judged by calculating GPT and GOT values. All types of hepatotoxicity are proportional to sGPT values. For this reason, lowering of sGPT is fundamental in the treatment of hepatic diseases [Cecil's textbook of medicine]. As an approach to prevent hepatotoxicity of drugs, there is a method of lowering sGPT to within a normal range by inhibiting the formation of lipid peroxides through the administration of an antioxidant or the like.

Inhibition of the formation of lipid peroxides in the liver should take place first on the cell membrane. This is because oxidative stress is concentrated on the cell membrane due to localization of lipids on the cell membrane. On the cell membrane, vitamin E removes peroxyl and hydroxyl radicals, thereby protecting the cell membrane against oxidative stress, and carotenoid which is vitamin A removes singlet oxygen. Vitamin E having an antioxidative action is regenerated by the action of vitamin C, the used vitamin C is regenerated by the action of glutathione, and glutathione is in turn regenerated by the action of cysteine. Therefore, the antioxidative action cascade requires antioxidative environment of the coupling system including vitamin E, vitamin C, glutathione and cysteine.

In addition to the above-stated antioxidants, there are oxidative stress-inhibiting ingredients among crude drug ingredients.

For example, silymarin or biphenyldimethyldicarboxylate (DDB) is a crude drug which has been well-known as an ingredient to counteract drug-induced hepatitis by inhibiting oxidative stress of cell membranes. Further, intrahepatic biliary atresia, one of pathological symptoms of drug-induced hepatitis, is a clinical condition leading to continuous destruction of surrounding hepatocytes due to no escape of cell lysates into the biliary duct. Although ursodeoxycholic acid exhibits surface-activating effects, not antioxidative effects, on such a drug-induced hepatic disorder, it is an ingredient having significant hepatic protective effects since ursodeoxycholic acid prevents worsening of pathological symptoms due to oxidative stress and increases the regeneration capacity of hepatocytes.

As reviewed above, with regard to long-term medication of an angiotensin-II-receptor blocker or various other drugs for the treatment of hypertension and prevention of related complications, there is a further need for the development of a formulation which is capable of preventing hepatic damage.

As a result of a variety of extensive and intensive studies and experiments to develop a pharmaceutical formulation which is capable of further enhancing drug efficacy upon administration of an angiotensin-II-receptor blocker when it is used as a single drug and which is capable of not only reducing adverse side effects but also further enhancing drug efficacy when concurrently administered with other drugs, the inventors of the present invention have completed the present invention. Further, the present inventors have conducted intensive studies and experiments to develop a pharmaceutical formulation which is excellent in patients' compliance and is capable of preventing drug interactions and preventing hepatic damage which may occur upon long-term administration, and thus the present invention has been completed.

### DISCLOSURE OF THE INVENTION

### TECHNICAL PROBLEM

Therefore, the present invention is intended to provide a formulation which is excellent in patients' compliance upon administration of an angiotensin-II-receptor blocker and is capable of preventing drug interaction and preventing hepatic damage which may occur upon long-term administration.

### TECHNICAL SOLUTION

The present invention provides a pharmaceutical formulation containing an angiotensin-II-receptor blocker and a release-controlling material.

The pharmacologically active ingredient angiotensin-II-receptor blocker in the formulation of the present invention is released at a level of less than 40% by weight within 4 hours after oral administration, and preferably a level of less than 30% by weight within 4 hours after oral administration.

A content of the angiotensin-II-receptor blocker in the formulation of the present invention is in the range of 2.5 to 1200 mg per unit formulation, and preferably 5 to 600 mg.

The formulation of the present invention further contains a hepatitis-preventing and inhibiting agent as a pharmacologically active ingredient, and a content of the hepatitis-preventing and inhibiting agent in the formulation is in the range of 0.01 µg to 1 g, and preferably 0.1 µg to 200 mg.

Examples of the hepatitis-preventing and inhibiting agent include vitamins such as β-carotene, riboflavin, riboflavin tetrabutyrate, riboflavin rocoat, riboflavin phosphate sodium, ascorbic acid, ascorbyl palmitate, calcium ascorbate, nicotinamide ascorbate, sodium ascorbate, dehydroascorbic acid, cholecalciferol, cholecalciferolic acid, ergocalciferol, tocopherol, tocopherol acetate, tocopherol calcium, tocopherol calcium succinate, and/or tocopherol succinate, amino acids such as cysteine, cysteine hydrochloride, cysteine malate, methyl cysteine, carboxymethyl cysteine, methyl cysteine hydrochloride, N-acetyl-L-cysteine, L-glutathione, glutathione disulfate, reduced glutathione, L-glutamine, glutamine hydrochloride, L-glutaminate-L-lysinate, and/or L-glutamine, N(2)-L-alanine-L-glutamine, α-lipoic acid, selenized yeast, selenium, selenium disulfide, sodium selenate, sodium selenite, silymarin, ginko biloba extract, DDB (biphenyldimethyldicarboxylate), ursodeoxycholic acid, chenocholic acid, butylated hydroxyanisole, butylated hydroxytoluene, guaiacol, erdosteine, resveratrol, pyridoxamine hydrochloride, pyridoxine hydrochloride, pyridoxal phosphate, agaro-oligosaccharide, fraction flavonoid purifiee micronise, melatonin, ubidecarenone, L-omithine, ornithine aspartate, ornithine hydrochloride, L-arginine, arginine hydrochloride, arginine sodium, L-arginine monoglutamate, protoporphyrin disodium, haematoporphyrin, haematoporphyrin hydrochloride, Coriolus versicolor polysaccharide (for example, ATSO: Agents for Liver and Gallbladder (Hepato protectors)), cicloxilic acid, citiolone, urazamide, azintamide, hymecromone, and/or α-naphthyl acetic acid, and the like. The hepatitis-preventing and inhibiting agent includes pharmaceutically acceptable salts thereof and derivatives and analogs thereof having an equivalent pharmacological activity.
The angiotensin-II-receptor blocker in the formulation of the present invention is at least one selected from losartan, valsartan, telmisartan, irbesartan, candesartan, olmesartan, eprosartan, isomers thereof, pharmaceutically acceptable salts thereof, and prodrugs thereof. The prodrug of candesartan is decomposed *in vivo* into an active ingredient candesartan and is preferably candesartan cilexetil. The prodrug of olmesartan is decomposed *in vivo* into an active ingredient olmesartan and is preferably olmesartan medoxomil.
The release-controlling material in the formulation of the present invention is at least one selected from an enteric polymer, a water-insoluble polymer, a hydrophobic compound, a hydrophilic polymer and a mixture thereof. The release-controlling material is preferably at least one selected from a water-insoluble polymer and an enteric polymer. A content of the release-controlling material is in the range of 0.05 to 30 parts by weight relative to 1 part by weight of the angiotensin-II-receptor blocker. If a content of the release-controlling material is lower than the above-specified range, it may be difficult to achieve a sufficient delayed-release property. On the other hand, if a content of the release-controlling material is higher than the above-specified range, significant clinical effects cannot be achieved due to delayed release of the drug.
A content of the release-controlling material in the formulation of the present invention is in the range of 0.01 to 100 parts by weight relative to 1 part by weight of the angiotensin-II-receptor blocker. If a content of the release-controlling material is lower than 0.01 parts by weight, it may be difficult to achieve a sufficient time-lag. On the other hand, if a content of the release-controlling material is higher than 100 parts by weight, it may be difficult to achieve significant clinical effects due to delayed release of the drug.

The enteric polymer refers to a polymer which is insoluble or stable under the acidic conditions of less than pH 5, and is dissolved or degraded under the specific pH conditions of pH 5 or higher. The enteric polymer that can be used in the present invention is at least one selected from the group consisting of an enteric cellulose derivative, an enteric acrylic acid copolymer, an enteric polymethacrylate copolymer, an enteric maleic acid copolymer, an enteric polyvinyl derivative, and a mixture thereof.

Preferably, the enteric cellulose derivative is at least one selected from hydroxypropylmethylcellulose acetate succinate (or referred to as hypromellose acetate succinate), hydroxypropylmethylcellulose phthalate (or referred to as hypromellose phthalate), hydroxymethylethylcellulose phthalate, cellulose acetate phthalate, cellulose acetate succinate, cellulose acetate maleate, cellulose benzoate phthalate, cellulose propionate phthalate, methylcellulose phthalate, carboxymethylethylcellulose, ethylhydroxyethylcellulose phthalate, methylhydroxyethylcellulose and a mixture thereof; the enteric acrylic acid copolymer is at least one selected from a styrene/acrylic acid copolymer, a methyl acrylate/acrylic acid copolymer, a methyl acrylate/methacrylic acid copolymer, a butyl acrylate/styrene/acrylic acid copolymer, a methyl acrylate/methacrylic acid/octyl acrylate copolymer and a mixture thereof; the enteric polymethacrylate copolymer is at least one selected from a poly(methacrylic acid/methyl methacrylate copolymer) (e.g., Eudragit L 100 or Eudragit S, Degussa, Germany), a poly(methacrylic acid/ethyl acrylate) copolymer (e.g., Eudragit L 100-55, Degussa, Germany) and a mixture thereof; the enteric maleic acid copolymer is at least one selected from a vinyl acetate/maleic anhydride copolymer, a styrene/maleic anhydride copolymer, a styrene/maleic monoester copolymer, a vinyl methyl ether/maleic anhydride copolymer, an ethylene/maleic anhydride copolymer, a vinyl butyl ether/maleic anhydride copolymer, an acrylonitrile/methyl acrylate/maleic anhydride copolymer, a butyl acrylate/styrene/maleic anhydride copolymer and a mixture thereof; and the enteric polyvinyl derivative is at least one selected from polyvinylalcohol phthalate, polyvinylacetate phthalate, polyvinylbutyrate phthalate, polyvinylacetacetal phthalate and a mixture thereof. The enteric polymer in the formulation of the present invention is preferably at least one selected from hydroxypropylmethylcellulose phthalate and a methacrylic acid/ethyl acrylate copolymer. In the formulation of the present invention, the enteric polymer is preferably hydroxypropylmethylcellulose phthalate or a methyl acrylate/methacrylic acid copolymer.

A content of the enteric polymer may be in the range of 0.1 parts by weight to 20 parts by weight, preferably 0.5 parts by weight to 10 parts by weight relative to 1 part by weight of the active ingredient. If a content of the enteric polymer is lower than 0.1 parts by weight, the enteric polymer may be easily dissolved at a pH of less than 5. On the other hand, if a content of the enteric polymer is higher than 20 parts by weight, this may lead to an unnecessary increase in a total weight of the formulation or excessively delayed release thereof.

The water-insoluble polymer refers to a pharmaceutically acceptable water-insoluble polymer which controls the release of a drug. The water-insoluble polymer that can be used in the present invention is preferably at least one selected from the group consisting of polyvinyl acetate, a water-insoluble polymethacrylate copolymer {e.g. poly(ethyl acrylate, methyl methacrylate) copolymer (such as Eudragit NE30D), a poly(ethyl acrylate, methyl methacrylate, trimethylaminoethyl methacrylate chloride) copolymer (e.g. Eudragit RS PO), etc.}, ethylcellulose, cellulose ester, cellulose ether, cellulose acylate, cellulose diacylate, cellulose triacylate, cellulose acetate, cellulose diacetate, cellulose triacetate and a mixture thereof, and more preferably at least one selected from polyvinyl acetate, ethylcellulose and cellulose acetate. A content of the water-insoluble polymer may be in the range of 0.1 parts by weight to 30 parts by weight, preferably 0.5 parts by weight to 20 parts by weight relative to 1 part by weight of the active ingredient. If a content of the water-insoluble polymer is lower than 0.1 parts by weight, release of the drug may be not controlled. On the other hand, if a content of the water-insoluble polymer is higher than 30 parts by weight, release of the drug may be excessively delayed.

The hydrophobic compound refers to a pharmaceutically acceptable water-insoluble material which controls the release of a drug. The hydrophobic compound that can be used in the present invention may be at least one selected from the group consisting of fatty acid and fatty acid ester, fatty acid alcohol, wax, inorganic material, and a mixture thereof. Preferably, the fatty acid or fatty acid ester is at least one selected from glyceryl palmitostearate, glyceryl stearate, glyceryl distearate, glyceryl behenate, cetyl palmitate, glyceryl monooleate, stearic acid and a mixture thereof; the fatty acid alcohol is at least one selected from cetostearyl alcohol, cetyl alcohol, stearyl alcohol and a mixture thereof; the wax is at least one selected from carnauba wax, beeswax, microcrystalline wax and a mixture thereof; and the inorganic material is at least one selected from talc, precipitated calcium carbonate, calcium hydrogen phosphate, zinc oxide, titanium oxide, kaolin, bentonite, montmorillonite, veegum and a mixture thereof . More preferred is carnauba wax.

A content of the hydrophobic compound may be in the range of 0.1 parts by weight to 20 parts by weight, preferably 0.5 parts by weight to 10 parts by weight relative to 1 part by weight of the active ingredient. If a content of the hydrophobic compound is lower than 0.1 parts by weight, release of the drug may be not controlled. On the other hand, if a content of the hydrophobic compound is higher than 20 parts by weight, release of the drug may be excessively delayed.

The hydrophilic polymer refers to a pharmaceutically acceptable water-soluble polymer which controls the release of a drug. The hydrophilic polymer that can be used in the present invention may be at least one selected from the group consisting of saccharide, a cellulose derivative, gum, a protein, a polyvinyl derivative, a hydrophilic polymethacrylate copolymer, a polyethylene derivative, a carboxyvinyl copolymer and a mixture thereof. Preferably, the saccharide is at least one selected from dextrin, polydextrin, dextran, pectin and a pectin derivative, alginate, polygalacturonic acid, xylan, arabinoxylan, arabinogalactan, starch, hydroxypropyl starch, amylose, amylopectin and a mixture thereof; the cellulose derivative is at least one selected from hydroxypropylmethylcellulose (or referred to as hypromellose), hydroxypropylcellulose, hydroxymethylcellulose, hydroxyethylcellulose, methylcellulose, sodium carboxymethylcellulose, hydroxypropylmethylcellulose acetate succinate, hydroxyethylmethylcellulose and a mixture thereof; the gum is at least one selected from guar gum, locust bean gum, tragacanth, carrageenan, gum acacia, gum arabic, gellan gum, xanthan gum and a mixture thereof; the protein is at least one selected from gelatin, casein, zein and a mixture thereof; the polyvinyl derivative is at least one selected from polyvinyl alcohol, polyvinyl pyrrolidone, polyvinylacetal diethylaminoacetate and a mixture thereof; the hydrophilic polymethacrylate copolymer is at least one selected from a poly(butyl methacrylate, (2-dimethylaminoethyl)methacrylate, methyl methacrylate) copolymer (e.g. Eudragit E100, Evonik, Germany) and a mixture thereof; the polyethylene derivative is at least one selected from polyethylene glycol, polyethylene oxide and a mixture thereof; and the carboxyvinyl polymer is carbomer. A preferred example of the hydrophilic polymer is at least one selected from polyvinylpyrrolidone, hydroxypropylcellulose, hypromellose, and a poly(ethyl acrylate, methyl methacrylate, triethylaminoethyl methacrylate chloride) copolymer.

A content of the hydrophilic polymer may be in the range of 0.05 parts by weight to 30 parts by weight, preferably 0.5 to 20 parts by weight relative to 1 part by weight of the active ingredient. If a content of the hydrophilic polymer is lower than 0.05 parts by weight, release of the drug may be not controlled. On the other hand, if a content of the hydrophilic polymer is higher than 30 parts by weight, release of the drug may be excessively delayed.
The formulation of the present invention may further contain, if necessary, commonly used additives such as pharmaceutically acceptable diluent, binder, disintegrant, lubricant, pH-adjusting agent, and solubilizer, within the range where effects of the present invention are not impaired and the release of pharmacologically active ingredients is not impaired.

In the formulation of the present invention, a content of the additive is in the range of 0.1 to 300 parts by weight, relative to 1 part by weight of the active ingredient.

Examples of the diluent that can be used in the present invention may include starch, microcrystalline cellulose, lactose, glucose, mannitol, alginate, an alkaline earth metal salt, clay, polyethylene glycol, dicalcium phosphate, and a mixture thereof.

Examples of the binder that can be used in the present invention may include starch, microcrystalline cellulose, highly dispersive silica, mannitol, sucrose, lactose, polyethylene glycol, polyvinylpyrrolidone, hydroxypropylmethylcellulose, hydroxypropylcellulose, natural gum, synthetic gum, a polyvinylpyrrolidone copolymer, povidone, gelatin, and a mixture thereof.

Examples of the disintegrant that can be used in the present invention may include starches or modified starches such as sodium starch glycolate, corn starch, potato starch, and pregelatinized starch; clays such as bentonite, montmorillonite, and veegum; celluloses such as microcrystalline cellulose, hydroxypropylcellulose, and carboxymethylcellulose; algins such as sodium alginate, and alginic acid; crosslinked celluloses such as croscarmellose sodium; gums such as guar gum, and xanthan gum; crosslinked polymers such as crosslinked polyvinylpyrrolidone (crospovidone); effervescent agents such as sodium bicarbonate and citric acid, and mixtures thereof.

Examples of the lubricant that can be used in the present invention may include talc, stearic acid, magnesium stearate, calcium stearate, sodium lauryl sulfate, hydrogenated vegetable oil, sodium benzoate, sodium stearyl fumarate, glyceryl behenate, glyceryl monolaurate, glyceryl monostearate, glyceryl palmitostearate, polyethylene glycol, and mixtures thereof.

Examples of the stabilizer that can be used in the present invention may include ascorbic acid, citric acid, butylated hydroxyanisole, butylated hydroxy toluene, and tocopherol derivatives. Further examples of the stabilizer may include alkalizers such as alkali metal salts, alkaline earth metal salts, or mixtures thereof. Preferably, there may be used calcium carbonate, sodium carbonate, sodium hydrogen carbonate, magnesium oxide, magnesium carbonate, and sodium citrate. Examples of the pH-adjusting agent that can be used in the present invention may include acidulants such as acetic acid, adipic acid, ascorbic acid, malic acid, succinic acid, tartaric acid, fumaric acid, and citric acid, and basifying agents such as precipitated calcium carbonate, aqueous ammonia, and meglumine.

Examples of the solubilizer that can be used in the present invention may include sodium lauryl sulfate, polyoxyethylene sorbitan fatty acid ester (such as polysorbate), and docusate sodium.

In addition, the formulation of the present invention may optionally contain pharmaceutically acceptable additives such as various additives selected from a colorant and a fragrance.
The range of the additive that can be used in the present invention is not limited to the above-mentioned additives, and the additive may be used in a conventional dose which can be suitably selected by those skilled in the art.
The formulation of the present invention can be prepared into various formulations. That is, the formulation of the present invention can be formulated into tablets (such as uncoated tablets, film-coated tablets, multi-layered tablets, and press-coated tablets), powders, granules, or capsules.

The uncoated tablet may be a tablet which is obtained by compression of granules containing an angiotensin-II-receptor blocker and a release-controlling material using a tablet press or the like.

The uncoated tablet can be prepared by coating a particle, granule or pellet containing an angiotensin-II-receptor blocker with a release-controlling material, followed by mixing with a pharmaceutically acceptable excipient(s) and compression into uniform weight.

The film-coated tablet may be a tablet in which a tablet containing an angiotensin-II-receptor blocker is coated with a film-coated layer containing a release-controlling material, using a coater or the like. The film-coated tablet can be prepared by compressing a particle, granule or pellet containing an angiotensin-II-receptor blocker into uniform weight, thereby preparing a tablet, and coating the resulting tablet with a release-controlling material, followed by drying to prepare a film-coated tablet, or coating the resulting film-coated tablet with a coating solution for the purpose of improving the stability, thereby preparing a film-coated tablet. Further, for the purpose of improving the stability or achieving delayed release of drugs, the compressed uncoated tablet of delayed-release granules may be coated with a coating solution, thereby preparing a film-coated tablet.
The multi-layered tablet may be a tablet which is formed to have a layered structure of a layer containing an angiotensin-II-receptor blocker and a release-controlling material and a layer containing a release-controlling material. The layer containing a release-controlling material can be formulated not so as to contain an angiotensin-II-receptor blocker. The multi-layered tablet can be compressed in the form of a double-layered tablet by adding a particle, granule or pellet coated with a release-controlling material containing an angiotensin-II-receptor blocker, a layer containing pharmaceutically acceptable excipient(s), and a layer (delayed-release layer) containing pharmaceutically acceptable excipient(s) together with a release-controlling material, using a multiple tablet press. If necessary, a triple or more multi-layered tablet may also be prepared by further adding another layer on the double-layered tablet. A coated multi-layered tablet may be prepared by coating the multi-layered tablet.
The press-coated tablet may be a tablet including an inner core tablet containing an angiotensin-II-receptor blocker and a release-controlling material and an outer layer containing a release-controlling material enclosing the outer surface of the inner core tablet. That is, the press-coated tablet may be a tablet including a coating layer enclosing the outer surface of the angiotensin-II-receptor blocker-containing tablet and containing a release-controlling material, and an outer layer enclosing the outer surface of the coating layer and containing a release-controlling material, or may be an osmotic press-coated tablet including an osmotic inner core tablet releasing the drug by means of osmotic pressure, as an inner core.

The capsule may be of a form where at least one selected from a particle, a granule, a pellet and a tablet is filled in a capsule. The capsule may be prepared by filling a granule containing an angiotensin-II-receptor blocker and coated with a release-controlling material, or optionally together with a particle or granule containing a pharmaceutically acceptable excipient and/or a release-controlling material, in a capsule using a capsule filling machine.

Further, the capsule may be prepared by filling a pellet coated with a release-controlling material containing an angiotensin-II-receptor blocker, or optionally together with a particle or granule containing a pharmaceutically acceptable excipient and/or a release-controlling material, in a capsule using a capsule filling machine.

Further, the capsule may be prepared by filling a film-coated tablet coated with a release-controlling material containing an angiotensin-II-receptor blocker, or optionally together with a particle or granule containing a pharmaceutically acceptable excipient and/or a release-controlling material, in a capsule using a capsule filling machine.

The formulation of the present invention may be a formulation in the form of an uncoated tablet without further coating, or otherwise, if necessary, may be provided in the form of a coated tablet further including a coating layer formed on the outside of the formulation. The formation of a coating layer can provide a formulation which is capable of further securing stability of pharmacologically active ingredients.
A method for forming the coating layer may be suitably selected by a skilled person in the art, from among methods capable of forming a film-like coating layer on the surface of the tablet layer, such as a fluidized-bed coating method and a pan coating method. Preferably, a pan coating method may be used.
The coating layer may be formed by using a film-forming agent, a film-forming aid or a mixture thereof. For example, the film-forming agent may be cellulose derivatives such as hydroxypropylmethylcellulose and hydroxypropylcellulose, saccharide derivatives, polyvinyl derivatives, waxes, fats, gelatin and mixtures thereof, and the film-forming aid may be polyethylene glycol, ethylcellulose, glyceride, titanium oxide, talc, diethyl phthalate and mixtures thereof.

A content of the coating layer may be in the range of 0.5 to 15% by weight (% w/w) based on the total weight of the tablet.

If a content of the coating layer is lower than 0.5% by weight, it may be difficult to achieve the protection of products, and the stability thereof depending on formulations. On the other hand, if a content of the coating layer is higher than 15% by weight, release profiles of pharmacologically active ingredients may be affected.

The formulation of the present invention may contain an osmo-regulator and may be coated with a semi-permeable membrane coating base.

The osmo-regulator is preferably at least one selected from the group consisting of magnesium sulfate, magnesium chloride, sodium chloride, lithium chloride, potassium sulfate, sodium sulfate, lithium sulfate and a mixture thereof.

A content of the osmo-regulator may be in the range of 0.01 parts by weight to 10 parts by weight, preferably 0.05 parts by weight to 0.5 parts by weight relative to 1 part by weight of the active ingredient. If a content of the osmo-regulator is lower than 0.01 parts by weight, it may be difficult to achieve a sufficient chronotherapeutic release. On the other hand, if a content of the osmo-regulator is higher than 10 parts by weight, it may be difficult to achieve significant clinical effects due to delayed release of the drug.

The semi-permeable membrane coating base is a material which is blended in a coating layer of the pharmaceutical formulation and refers to a material used to form a membrane through which some ingredients can pass but other ingredients cannot pass. The semi-permeable coating base in the present invention may employ the above-mentioned water-insoluble polymers.
For example, the semi-permeable membrane coating base that can be used in the present invention is at least one selected from the group consisting of polyvinyl acetate, a polymethacrylate copolymer, poly(ethyl acrylate, methyl methacrylate) copolymer, a poly(ethyl acrylate, methyl methacrylate, trimethylaminoethyl methacrylate chloride) copolymer, ethylcellulose, cellulose ester, cellulose ether, cellulose acylate, cellulose diacylate, cellulose triacylate, cellulose acetate, cellulose diacetate, cellulose triacetate and a mixture thereof.
A content of the semi-permeable membrane coating base may be in the range of 0.01 parts by weight to 10 parts by weight, preferably 0.05 parts by weight to 1.25 parts by weight relative to 1 part by weight of the active ingredient. If a content of the semi-permeable membrane coating base is lower than 0.01 parts by weight, it may be difficult to achieve a sufficient time-lag. On the other hand, if a content of the semi-permeable membrane coating base is higher than 10 parts by weight, there is a problem associated with no release of the drug or an excessively long time-lag.

The formulation of the present invention may be administered once a day, between 5 p.m. and 11 p.m. and may be administered in combination with a drug which is metabolized by cytochrome.

Examples of the drug which is metabolized by cytochrome include a lipid inhibitor which is at least one selected from simvastatin, and atorvastatin; an antihypertensive agent which is at least one selected from amlodipine, felodipine, carvedilol, and aliskiren; an antihistamine agent which is at least one selected from loratadine, and azelastine; an analgesic agent which is at least one selected from acetaminophen, and tramadol; an antidepressant which is at least one selected from amitriptyline, and imipramine; an anticoagulant which is warfarin; an anti-emetic agent which is at least one selected from chlorpromazine, and granisetron; an anticonvulsant which is carbamazepine; an anti-acne agent (retinoid) which is isotretinoin; an antipsychotic agent which is risperidone; an antidepressant which is at least one selected from propyne, and venlafaxine; a human immunodeficiency virus (HIV)-antiviral agent (protease inhibitor) which is fosamprenavir; an antifungal agent which is itraconazole; a thiazolidinedione antidiabetic agent which is pioglitazone; an anti-obesity agent which is sibutramine; an anti-erectile dysfunction agent which is sildenafil; and an anti-incontinence agent which is tolterodine.

Further, the present invention provides a pharmaceutical formulation including a prior-release compartment containing a hepatitis-preventing and inhibiting agent as a pharmacologically active ingredient, and a delayed-release compartment containing an angiotensin-II-receptor blocker as a pharmacologically active ingredient.

Preferably, the pharmacologically active ingredient contained in the delayed-release compartment is released 1 to 4 hours after the release of the pharmacologically active ingredient contained in the prior-release compartment is initiated. Less than 40% by weight of the pharmacologically active ingredient contained in the delayed-release compartment is released within 4 hours after the release of the pharmacologically active ingredient contained in the prior-release compartment is initiated.

Further, preferably, the pharmacologically active ingredient contained in the prior-release compartment is released at a level of more than 85% by weight within one hour after initiation of release thereof.

A content of the hepatitis-preventing and inhibiting agent in the formulation of the present invention is in the range of 0.01 µg to 1 g.

A content of the angiotensin-II-receptor blocker in the formulation of the present invention is in the range of 5 to 600 mg.

Hereinafter, individual compartments of the formulation in accordance with the present invention will be described in more detail.

### 1. Prior-release compartment

The "prior-release compartment" refers to a compartment which is released ahead of the "delayed-release compartment" in the pharmaceutical formulation of the present invention. The prior-release compartment may further contain "pharmaceutically acceptable additives", if necessary, in addition to "pharmacologically active ingredients".

### (1) Pharmacologically active ingredients

The pharmacologically active ingredient of the prior-release compartment is a hepatitis-preventing and inhibiting agent which is the same as described in the pharmaceutical formulation containing an angiotensin-II-receptor blocker and a release-controlling material.

### (2) Pharmaceutically acceptable additives

The formulation of the present invention may further contain commonly used additives such as pharmaceutically acceptable diluent, binder, disintegrant, lubricant, pH-adjusting agent, and solubilizer, within the range where effects of the present invention are not impaired and the release of pharmacologically active ingredients is not impaired. Details of the pharmaceutically acceptable additives are the same as those of the pharmaceutical formulation containing an angiotensin-II-receptor blocker and a release-controlling material.

### 2. Delayed-release compartment

In the present invention, the "delayed-release compartment" refers to a compartment whose active ingredient is released at a certain time interval after the release of the active ingredient of the "prior-release compartment". The delayed-release compartment may contain (1) a "pharmacologically active ingredient" and (2-1) a "release-controlling material" or (2-2) an osmo-regulator and a semi-permeable membrane coating base, and (3), if necessary, "pharmaceutically acceptable additives".

### (1) Pharmacologically active ingredients

The pharmacologically active ingredient of the delayed-release compartment is an angiotensin-II-receptor blocker and may further include the hepatitis-preventing and inhibiting agent of the prior-release compartment, if necessary. The pharmacologically active ingredient of the delayed-release compartment in the formulation of the present invention is released at a level of less than 40% by weight of a total amount of the active ingredient of the delayed-release compartment up to 2 hours after the release of the pharmacologically active ingredient of the prior-release compartment is initiated. Preferably, the pharmacologically active ingredient of the delayed-release compartment is released at a level of less than 20% by weight of a total amount of the pharmacologically active ingredient of the delayed-release compartment up to 2 hours after the release of the pharmacologically active ingredient of the prior-release compartment is initiated.

Details of the angiotensin-II-receptor blocker and the hepatitis-preventing and inhibiting agent are the same as those of the pharmaceutical formulation containing an angiotensin-II-receptor blocker and a release-controlling material.

### (2-1) Release-controlling materials

The delayed-release compartment in the pharmaceutical formulation of the present invention contains a release-controlling material. Details of the release-controlling material are the same as those of the pharmaceutical formulation containing an angiotensin-II-receptor blocker and a release-controlling material, provided that there is a difference in the type of compartments and formulations.

### (2-2) Osmo-regulator and semi-permeable membrane coating base

The delayed-release compartment of the present invention may be a compartment which contains an osmo-regulator and is coated by a semi-permeable membrane coating base.

Details of the osmo-regulator and semi-permeable membrane coating base are the same as those of the pharmaceutical formulation containing an angiotensin-II-receptor blocker and a release-controlling material.

### (3) Pharmaceutically acceptable additives

In addition to (2-1) a release-controlling material and (2-2) an osmo-regulator and a semi-permeable membrane coating base, the formulation of the present invention may further contain commonly used additives such as pharmaceutically acceptable diluent, binder, disintegrant, lubricant, pH-adjusting agent, anti-foaming agent, and solubilizer, within the range where the effects of the present invention are not impaired and within the range where delayed-release properties are not compromised.

Details of the pharmaceutically acceptable additives are the same as those of the pharmaceutical formulation containing an angiotensin-II-receptor blocker and a release-controlling material.

The pharmaceutical formulation of the present invention can be prepared into various formulations, for example, tablets (such as uncoated tablets, coated tablets, multi-layered tablets, and press-coated tablets), powders, granules, or capsules.

The formulation of the present invention may be in the form of an uncoated tablet which is obtained by optionally subjecting prior-release compartment-forming granules or the like and delayed-release compartment-forming granules or the like to post-mixing with additive(s), followed by compression, such that the prior-release compartment and the delayed-release compartment are present within a single tablet whereby active ingredients of individual compartments are separately released to exhibit the efficacy of each drug.

The formulation of the present invention may be in the form of a two-phase matrix tablet which is obtained by uniformly mixing a delayed-release compartment and a prior-release compartment, followed by compression.
Further, the pharmaceutical formulation of the present invention may be in the form of a film-coated tablet including a tablet consisting of a delayed-release compartment and a film-coated layer consisting of a prior-release compartment enclosing the exterior of the tablet, whereby an active ingredient of the film-coated layer is first released as the film-coated layer is dissolved.

Further, the pharmaceutical formulation of the present invention may be in the form of a multi-layered tablet having a multi-layered structure of a delayed-release compartment and a prior-release compartment, each compartment of which is obtained by mixing the granules constituting the delayed-release compartment and the prior-release compartment with pharmaceutical additives, and compressing the mixture into a double-layered or triple-layered tablet, using a multiple tablet press. The resulting formulation is a tablet for oral administration which was formulated to achieve the prior-release and delayed-release of drugs according to individual layers.

Further, the pharmaceutical formulation of the present invention may be in the form of a press-coated tablet including an inner core tablet consisting of a delayed-release compartment and an outer layer consisting of a prior-release compartment enclosing the outer surface of the inner core tablet. The press-coated tablet may be an osmotic press-coated tablet. The osmotic press-coated tablet is a formulation wherein the tablet mix is compressed into a tablet in a manner that an osmo-regulator is incorporated for the delayed-release of a drug, the tablet surface is coated with a semi-permeable membrane coating base to prepare an inner core, a granule constituting the prior-release compartment is mixed with pharmaceutical additives to prepare an outer layer, followed by compression to form a formulation having a delayed-release inner core and a prior-release layer enclosing the surface of the inner core.

The pharmaceutical formulation of the present invention may be in the form of a capsule containing a particle, granule, pellet, or tablet formed of a delayed-release compartment and a particle, granule, pellet, or tablet formed of a prior-release compartment.

The tablet formed of the delayed-release compartment of the capsule may be an osmotic coated tablet which contains an osmo-regulator inside the tablet and has a semi-permeable membrane coating base on the surface of the tablet.

The base material of the capsule may be one selected from gelatin, succinate gelatin, hydroxypropylmethylcellulose, and a mixture thereof.
Further, the pharmaceutical formulation of the present invention may be in the form of a kit including a delayed-release compartment and a prior-release compartment. Specifically, the kit includes (a) prior-release compartment; (b) a delayed-release compartment; and (c) a container for filling the prior-release compartment and the delayed-release compartment. The kit can be prepared in the form of a kit wherein a particle, granule, pellet, or tablet constituting the prior-release compartment is prepared, a granule, pellet, or tablet constituting the delayed-release compartment is separately prepared, and the thus prepared two compartment materials are filled in a foil, blister, or bottle to prepare a dosage form for concurrent administration of different drugs.
The formulation of the present invention may further include a coating layer on the outside of the delayed-release compartment and/or the prior-release compartment. That is, the surface of particles, granules, pellets, or tablets formed of the delayed-release compartment and/or the prior-release compartment may be coated for the purpose of controlled release of drugs or stability of the formulation.
Further, the formulation of the present invention may be a formulation in the form of an uncoated tablet without further coating, or otherwise, if necessary, may be provided in the form of a coated tablet further including a coating layer formed on the outside of the formulation. The formation of a coating layer can provide a formulation which is capable of further securing the stability of pharmacologically active ingredients. Details of the coating are the same as those of the pharmaceutical formulation containing an angiotensin-II-receptor blocker and a release-controlling material.
The formulation of the present invention may be administered once a day, between 5 p.m. and 11 p.m. and may be administered in combination with a drug which is metabolized by cytochrome. Details thereof are the same as those of the pharmaceutical formulation containing an angiotensin-II-receptor blocker and a release-controlling material.

The pharmaceutical formulation of the present invention can be preferably formulated into a desired dosage form depending on individual diseases or ingredients, by an appropriate method known in the art, for example, using the principle of the chronotherapy as disclosed in Chronotherapeutics (2003, Peter Redfern, PhP), specifically by a method including the following steps.
Step 1 is a step of obtaining a delayed-release granule or tablet by subjecting a pharmacologically active ingredient of the delayed-release compartment and one or two release-controlling materials selected from the group consisting of an enteric polymer, a water-insoluble polymer, a hydrophobic compound, and a hydrophilic polymer together with a pharmaceutically acceptable conventional additive to mixing, kneading, drying, granulation or coating, and compression, or of obtaining a delayed-release granule or tablet by subjecting the pharmacologically active ingredient and an osmo-regulator together with a conventional pharmaceutically acceptable additive to mixing, kneading, drying, granulation or compression, followed by coating with a semi-permeable membrane coating base.
Step 2 is a step of obtaining a prior-release granule or tablet by subjecting a pharmacologically active ingredient of the prior-release compartment together with a conventional pharmaceutically acceptable additive to conventional processes for producing oral solid formulations, for example, mixing, kneading, drying, granulation or coating, and compression.

Step 3 is a step of obtaining a formulation for oral administration by mixing the granule or tablet obtained in each of Steps 1 and 2 with a pharmaceutically acceptable excipient and either compressing the mixture into a tablet or filling the mixture in a capsule for oral administration.

Step 1 may be carried out after Step 2, or Step 1 may be carried out simultaneously with Step 2.

The pharmaceutical formulation of the present invention can be prepared according to the above procedure, and a formulation method of Step 3 will be described in more detail hereinafter, but the present invention is not limited thereto.

### 1. Preparation of two-phase matrix tablets

The particles or granules prepared in Step 1 are optionally coated with a release-controlling material and then mixed with the granules prepared in Step 2, followed by compression into uniform weight, thereby preparing tablets. The resulting tablets may be film-coated for the purpose of improving the stability or shape, if necessary.

### 2. Preparation of film-coated tablets containing pharmacologically active ingredients

The coated tablets or granules prepared in Step 1 are optionally coated with a release-controlling material and dried, followed by compression into uniform weight and optionally further coating to prepare tablets. In addition, a pharmacologically active ingredient of the prior-release compartment is dissolved and dispersed in a water-soluble film coating solution and is coated on the outer layer of the tablets obtained in Step 1 to thereby prepare oral film-coated tablets containing pharmacologically active ingredients in the film coating.

### 3. Preparation of multi-layered tablets

The granules prepared in Step 1 are optionally coated with a release-controlling material, and dried. The dried granules are compressed with the granules prepared in Step 2 by using a multi-layered tablet press, thereby obtaining a double-layered tablet. According to the formulation design or if necessary, a triple or more multi-layered tablet may also be prepared by further adding a release adjuvant layer on the double-layered tablet. A coated multi-layered tablet may be prepared by coating the multi-layered tablet.

### 4. Preparation of press-coated tablets

The coated tablets or granules prepared in Step 1 are optionally coated with a release-controlling material and dried, followed by compression into uniform weight. The resulting granules are used as an inner core optionally after performing further coating, and compressed with the granules prepared in Step 2 by using a press-coated tablet press, thereby providing press-coated tablets where the surface of the tablet of Step 1 is enclosed by the prior-release layer. Coated press-coated tablets may be prepared by coating the press-coated tablets.

### 5. Preparation of capsules (containing granules or tablets)

The granules prepared in Step 1 are optionally coated with a release-controlling material, and dried. The dried granules together with the granules prepared in Step 2 may be placed in a capsule filling machine, and filled in a capsule having a given size at an effective amount of each main ingredient, thereby preparing a capsule.

### 6. Preparation of capsules (pellets)

(1) A pharmacologically active ingredient of the delayed-release compartment, a release-controlling material, and if necessary, pharmaceutically acceptable additives are dissolved or suspended in water, an organic solvent, or a mixed solvent. This solution or suspension is coated on sugar spheres and dried, and if necessary, coated with one or more release-controlling materials dissolved in water, an organic solvent, or a mixed solvent, followed by drying. The mixture may be mixed with the granules prepared in Step 2 or the tablets obtained in Step 3 and then filled in capsules using a capsule filling machine, thereby preparing capsules.
(2) A pharmacologically active ingredient of the prior-release compartment and pharmaceutically acceptable additives may be dissolved or suspended in water, an organic solvent or a mixed solvent, coated on sugar spheres, followed by drying, and mixed with the controlled-release pellets of Section (1) containing a pharmacologically active ingredient of the delayed-release compartment and filled in capsules using a capsule filling machine to prepare capsules.
The formulation of the present invention is designed based on Chronotherapy taking into consideration the expression time of pharmacological action *in vivo,* and maximizes pharmacological and clinical hypotensive effects and complication-preventing effects upon administration thereof by the formulation such that release of the drug is delayed as compared to administration of an angiotensin-II-receptor blocker single drug which is immediately released *in vivo* after administration thereof. Further, the pharmaceutical formulation of the present invention is designed based on Xenobiotics taking into consideration the metabolic enzyme interaction, and maximizes pharmacological and clinical hypotensive effects and complication-preventing effects and further reduces adverse side effects by the formulation such that release of the drug is delayed as compared to a drug metabolized by hepatic cytochrome, thus providing a time-lag interval of release between different drugs without causing competitiveness therebetween on the metabolism by the same enzyme cytochrome in the liver. Further, the pharmaceutical formulation of the present invention can prevent and inhibit the onset of drug-induced hepatitis due to long-term administration or excessive administration.

### ADVANTAGEOUS EFFECTS

The formulation of the present invention maximizes the effectiveness on pharmacologically and clinically lowering blood pressure and preventing complications when taking the formulation, helps to avoid interactions with a drug which is metabolized by the same enzyme in the liver, and prevents and inhibits the incidence of drug-induced hepatitis which is caused by drug administration for a long time.

### DESCRIPTION OF THE DRAWINGS

FIG. 1 is a graph showing the dissolution profiles for a telmisartan single drug and a formulation of Example 1.
FIG. 2 is a graph showing the dissolution profiles for a losartan single drug and a formulation of Example 5.
FIG. 3 is a graph showing the dissolution profiles for formulations of Examples 8 to 11.
FIG. 4 is a graph showing the comparative dissolution profiles for formulations of Examples 10, and 12 to 14.
FIG. 5 is a graph showing the dissolution profiles for a valsartan single drug and a formulation of Example 32.
FIG. 6 is a graph showing the dissolution profiles for a candesartan single drug and a formulation of Example 35.
FIG. 7 is a graph showing the dissolution profiles for an olmesartan medoxomil single drug and a formulation of Example 40.
FIG. 8 is a graph showing the dissolution profiles of an eprosartan single drug and a formulation of Example 43.
FIG. 9 is a graph showing the dissolution profiles for an irbesartan single drug and a formulation of Example 45.

### MODE FOR INVENTION

Now, the present invention will be described in more detail with reference to the following Examples. These examples are provided only for illustrating the present invention and should not be construed as limiting the scope and spirit of the present invention.

### <Example 1> Preparation of angiotensin-II-receptor blocker-containing uncoated tablets

### 1) Preparation of angiotensin-II-receptor blocker delayed-release granules

According to the ingredient contents shown in Table 1 below, telmisartan, microcrystalline cellulose, sorbitol, meglumine, and sodium hydroxide were sieved through a No. 35 sieve and mixed in a high-speed mixer for 5 minutes to prepare a mixture. Meanwhile, polyvinylpyrrolidone was dissolved in purified water (70 mg/tablet) to prepare a binding solution, followed by kneading, granulation and drying. The dried material was placed in a fluidized bed coater. Meanwhile, polyvinyl acetate was dissolved in a (2:8) mixed solvent of ethanol and methylene chloride (240 mg/tablet) to prepare a solution which was then coated on the granules in a fluidized bed coater (GPCG-1: Glatt, Germany). After completion of the coating process, drying was carried out to prepare angiotensin-II-receptor blocker delayed-release granules.

### 2) Compression

The angiotensin-II-receptor blocker delayed-release granules prepared in Section 1) and magnesium stearate were placed in a double cone mixer, followed by final mixing for 4 minutes. The final mixture was compressed into tablets using a rotary tablet press (MRC-33: Sejong, South Korea) to prepare delayed-release uncoated tablets of the angiotensin-II-receptor blocker.

### <Example 2> Preparation of angiotensin-11-receptor blocker-containing uncoated tablets

### 1) Preparation of angiotensin-11-receptor blocker delayed-release granules

According to the ingredient contents shown in Table 1 below, irbesartan, pregelatinized starch, lactose, and croscarmellose sodium were sieved through a No. 35 sieve and mixed in a high-speed mixer for 5 minutes to prepare a mixture. Meanwhile, Poloxamer 188 was dissolved in purified water (90 mg/tablet) to prepare a binding solution, followed by kneading, granulation and drying. The dried material was placed in a fluidized bed coater. Meanwhile, cellulose acetate (acetyl group 32.0%) and cellulose acetate (acetyl group 39.8%) were dissolved in a (2:8) mixed solvent of ethanol and methylene chloride (800 mg/tablet) to prepare a solution which was then coated on the granules in a fluidized bed coater (GPCG-1: Glatt, Germany). After completion of the coating process, drying was carried out. The dried material, microcrystalline cellulose, and colloidal silicon dioxide were placed in a double cone mixer, followed by mixing to prepare angiotensin-II-receptor blocker delayed-release granules.

### 2) Compression

The angiotensin-II-receptor blocker delayed-release granules prepared in Section 1) and magnesium stearate were placed in a double cone mixer, followed by final mixing for 4 minutes. The final mixture was compressed into tablets using a rotary tablet press (MRC-33: Sejong, South Korea), thereby preparing delayed-release uncoated tablets of the angiotensin-II-receptor blocker.

### <Example 3> Preparation of angiotensin-II-receptor blocker-containing uncoated tablets

### 1) Preparation of angiotensin-II-receptor blocker delayed-release granules

According to the ingredient contents shown in Table 1 below, valsartan, microcrystalline cellulose, and crosslinked polyvinylpyrrolidone were sieved through a No. 35 sieve and mixed in a high-speed mixer for 5 minutes to prepare a mixture. Meanwhile, polyvinylpyrrolidone was dissolved in purified water (100 mg/tablet) to prepare a binding solution, followed by kneading, granulation and drying. The dried material was placed in a fluidized bed coater. Meanwhile, a solution of hypromellose in purified water (70 mg/tablet) and a solution of hypromellose phthalate in a (8:2) mixed solvent of ethanol and purified water (120 mg/tablet) were respectively prepared. The granules were placed in a fluidized bed coater (GPCG-1: Glatt, Germany), followed by two-step coating with the prepared solutions. After completion of the coating process, drying was carried out to prepare angiotensin-II-receptor blocker delayed-release granules.

### 2) Compression

The angiotensin-II-receptor blocker delayed-release granules prepared in Section 1) and magnesium stearate were placed in a double cone mixer, followed by final mixing for 4 minutes. The final mixture was compressed into tablets using a rotary tablet press (MRC-33: Sejong, South Korea), thereby preparing delayed-release uncoated tablets of the angiotensin-II-receptor blocker.

### <Example 4> Preparation of angiotensin-II-receptor blocker-containing uncoated tablets

### 1) Preparation of angiotensin-II-receptor blocker delayed-release granules

According to the ingredient contents shown in Table 1 below, losartan potassium, microcrystalline cellulose, sodium starch glycolate, and lactose were sieved through a No. 35 sieve and mixed in a high-speed mixer for 5 minutes to prepare a mixture. Meanwhile, hydroxypropylcellulose was dissolved in purified water (40 mg/tablet) to prepare a binding solution, followed by kneading, granulation and drying. The dried material was placed in a fluidized bed coater. Meanwhile, hypromellose phthalate (HP-55) and polyethylene glycol 6000 were dissolved in a (2:8) mixed solvent of ethanol and methylene chloride (1200 mg/tablet) to prepare a solution which was then coated on the granules in a fluidized bed coater (GPCG-1: Glatt, Germany). After completion of the coating process, drying was carried out. The dried material and colloidal silicon dioxide were placed in a double cone mixer, followed by mixing to prepare angiotensin-II-receptor blocker delayed-release granules.

### 2) Compression

The angiotensin-II-receptor blocker delayed-release granules prepared in Section 1) and magnesium stearate were placed in a double cone mixer, followed by final mixing for 4 minutes. The final mixture was compressed into tablets using a rotary tablet press (MRC-33: Sejong, South Korea), thereby preparing delayed-release uncoated tablets of the angiotensin-II-receptor blocker.

### <Example 5> Preparation of film-coated tablets containing delayed-release granules

### 1) Preparation of angiotensin-II-receptor blocker delayed-release uncoated tablets

Angiotensin-II-receptor blocker delayed-release uncoated tablets were prepared in the same manner as in Sections 1) and 2) of Example 4 and according to the ingredient compositions and contents shown in Table 1 below.

### 2) Film coating

The uncoated tablets prepared in Section 1) were placed in a Hi-coater (SFC-30N, Sejong Machinery Co., Ltd., South Korea). According to the ingredient compositions shown in Table 1 below, hypromellose 2910, hydroxypropylcellulose, titanium oxide, and talc were dissolved and dispersed in a (8:2) mixed solvent of ethanol and purified water (400 mg/tablet) to prepare a coating solution which was then coated on the uncoated tablets to prepare angiotensin-II-receptor blocker-containing film-coated tablets.

### <Example 6> Preparation of film-coated tablets containing delayed-release granules

### 1) Preparation of angiotensin-II-receptor blocker delayed-release uncoated tablets

Angiotensin-II-receptor blocker delayed-release uncoated tablets were prepared in the same manner as in Sections 1) and 2) of Example 4 and according to the ingredient compositions and contents shown in Table 1 below, except that eprosartan was used in place of losartan potassium.

### 2) Film coating

Film-coated tablets containing delayed-release granules were prepared in the same manner as in the preparation of a film-coated layer in Section 2) of Example 5 and according to the ingredient compositions and contents shown in Table 1 below.

### <Example 7> Preparation of film-coated tablets containing delayed-release granules

### 1) Preparation of angiotensin-II-receptor blocker delayed-release uncoated tablets

Angiotensin-II-receptor blocker delayed-release uncoated tablets were prepared in the same manner as in Sections 1) and 2) of Example 1 and according to the ingredient compositions and contents shown in Table 1 below.

### 2) Film coating

Film-coated tablets containing delayed-release granules were prepared in the same manner as in the preparation of a film-coated layer in Section 2) of Example 5 and according to the ingredient compositions and contents shown in Table 1 below.

### <Examples 8 to 11> Preparation of film-coated tablets containing a delayed-release film

### 1) Preparation of angiotensin-II-receptor blocker uncoated tablets

In the case of Example 8, for the preparation of angiotensin-II-receptor blocker delayed-release film-coated tablets, according to the ingredient compositions shown in Table 2 below, losartan potassium, microcrystalline cellulose, pregelatinized starch, a polyvinylpyrrolidone copolymer, and colloidal silicon dioxide were sieved through a No. 35 sieve and mixed in a double cone mixer for 30 minutes to prepare a mixture. To the mixture was added magnesium stearate, followed by mixing for 4 minutes. The final mixture was compressed into tablets using a rotary tablet press (MRC-33: Sejong, South Korea), thereby preparing angiotensin-II-receptor blocker uncoated tablets.

In the case of Examples 9 to 11, angiotensin-II-receptor blocker uncoated tablets were prepared in the same manner as above and according to the ingredient compositions and contents shown in Table 1 below.

### 2) Preparation of angiotensin-II-receptor blocker delayed-release film-coated tablets

The uncoated tablets prepared in Section 1) were placed in a Hi-coater (SFC-30N, Sejong Machinery Co., Ltd., South Korea). According to the ingredient contents shown in Table 2 below, ethylcellulose and Acryl-EZE were dissolved and dispersed in purified water (80 mg/tablet) to prepare a coating solution which was then coated thereon to prepare angiotensin-II-receptor blocker delayed-release film-coated tablets containing a delayed-release film.

### 3) Film coating containing a delayed-release film

The delayed-release film-coated tablets prepared in Section 2) were placed in a Hi-coater (SFC-30N, Sejong Machinery Co., Ltd., South Korea) and coated in the same manner as in the preparation of a film-coated layer in Section 2) of Example 5 and according to the ingredient compositions and contents shown in Table 1 below, thereby preparing film-coated tablets containing a delayed-release film.

### <Examples 12 to 14> Preparation of film-coated tablets containing a delayed-release film

### 1) Preparation of angiotensin-II-receptor blocker uncoated tablets

In the case of Examples 12 to 14, angiotensin-II-receptor blocker uncoated tablets were prepared in the same manner as in Section 1) of Example 8 and according to the ingredient compositions and contents shown in Table 2 below.

### 2) Preparation of angiotensin-II-receptor blocker delayed-release film-coated tablets

In the case of Examples 12 to 14, the uncoated tablets prepared in Section 1) were placed in a Hi-coater (SFC-30N, Sejong Machinery Co., Ltd., South Korea). According to the ingredient contents shown in Table 2 below, polyvinylpyrrolidone, ethylcellulose and Acryl-EZE were dissolved and dispersed in purified water (80 mg/tablet) to prepare a coating solution which was then coated thereon to prepare angiotensin-II-receptor blocker delayed-release film-coated tablets containing a delayed-release film.

### 3 Film coating containing a delayed-release film

In the case of Examples 12 to 14, the delayed-release film-coated tablets prepared in Section 2) were placed in a Hi-coater (SFC-30N, Sejong Machinery Co., Ltd., South Korea), and coated in the same manner as in the preparation of a film-coated layer in Section 2) of Example 5 and according to the ingredient compositions and contents shown in Table 1 below, thereby preparing film-coated tablets containing a delayed-release film.

### <Example 15> Preparation of capsules (granules)

### 1) Preparation of angiotensin-II-receptor blocker delayed-release granules

According to the ingredient contents shown in Table 3 below, olmesartan medoxomil, microcrystalline cellulose, and pregelatinized starch were sieved through a No. 35 sieve and mixed in a high-speed mixer for 5 minutes to prepare a mixture. Meanwhile, hydroxypropylcellulose was dissolved in purified water (30 mg/tablet) to prepare a binding solution, followed by kneading, granulation and drying. The dried material was placed in a fluidized bed coater. Meanwhile, polyvinyl acetate was dissolved in a (2:8) mixed solvent of ethanol and methylene chloride (240 mg/tablet) to prepare a solution which was then coated on the granules in a fluidized bed coater (GPCG-1: Glatt, Germany) to prepare angiotensin-II-receptor blocker delayed-release granules.

### 2) Mixing and capsule filling

To the granules prepared in Section 1) was added magnesium stearate, followed by final mixing in a double cone mixer. The final mixture was placed in a powder feeder and then filled in capsules using a capsule filling machine, thereby preparing a delayed-release formulation in the form of a capsule.

### <Example 16> Preparation of capsules (pellets)

### 1) Preparation of angiotensin-II-receptor blocker delayed-release pellets

Sugar seeds (sugar sphere) were sieved through a No. 35 sieve and placed in a fluidized bed granulator (GPCG 1: Glatt). Meanwhile, hypromellose and losartan potassium were dissolved in a (8:2) mixed solvent of ethanol and purified water (750 mg/tablet) to prepare a binding solution which was then sprayed thereon to form angiotensin-II-receptor blocker-containing pellets, followed by drying. A solution of hypromellose phthalate (HP-55) in a (2:8) mixed solvent of ethanol and methylene chloride (1200 mg/tablet) was sprayed on the granules to prepare angiotensin-II-receptor blocker delayed-release pellets.

### 2) Mixing and capsule filling

To the pellets prepared in Section 1) was added magnesium stearate, followed by final mixing in a double cone mixer. The final mixture was placed in a powder feeder and then filled in capsules using a capsule filling machine, thereby preparing a formulation in the form of a capsule.

### <Example 17> Preparation of capsules (tablets)

### 1) Preparation of angiotensin-II-receptor blocker delayed-release uncoated tablets

Angiotensin-II-receptor blocker delayed-release uncoated tablets were prepared in the same manner as in Section 1) of Example 8 and according to the ingredient compositions and contents shown in Table 3 below.

### 2) Film coating containing a delayed-release film

The uncoated tablets prepared in Section 1) were placed in a Hi-coater (SFC-30N, Sejong Machinery Co., Ltd., South Korea). According to the ingredient contents shown in Table 3 below, hypromellose phthalate (HP-55) and polyethylene glycol 6000 were dissolved and dispersed in a (8:2) mixed solvent of ethanol and purified water (80 mg/tablet) to prepare a coating solution which was then coated thereon to prepare angiotensin-II-receptor blocker film-coated tablets containing a delayed-release film.

### 3) Mixing and capsule filling

The final product of Section 2) was placed in a powder feeder and then filled in capsules using a capsule filling machine, thereby preparing a formulation in the form of a capsule.

### <Example 18> Preparation of uncoated tablets containing a hepatitis-preventing and inhibiting agent and an angiotensin-II-receptor blocker

### 1) Preparation of delayed-release granules

According to the ingredient contents shown in Table 3 below, telmisartan, α-lipoic acid, microcrystalline cellulose, sorbitol, and meglumine were sieved through a No. 35 sieve and mixed in a high-speed mixer for 5 minutes to prepare a mixture. Meanwhile, polyvinylpyrrolidone and sodium hydroxide were dissolved in purified water (70 mg/tablet) to prepare a binding solution, followed by kneading, granulation and drying. The dried material was placed in a fluidized bed coater. Meanwhile, polyvinyl acetate was dissolved in a (2:8) mixed solvent of ethanol and methylene chloride (1200 mg/tablet) to prepare a solution which was then coated on the granules in a fluidized bed coater (GPCG-1: Glatt, Germany). After completion of the coating process, drying was carried out to prepare desired granules.

### 2) Compression

The delayed-release granules prepared in Section 1) and magnesium stearate at the content shown in Table 3 below were placed in a double cone mixer, followed by final mixing for 4 minutes. The final mixture was compressed into tablets using a rotary tablet press (MRC-33: Sejong, South Korea), thereby preparing uncoated tablets containing α-lipoic acid and telmisartan.

### <Example 19> Preparation of uncoated tablets containing a hepatitis-preventing and inhibiting agent and an angiotensin-II-receptor blocker

### 1) Preparation of delayed-release granules

According to the ingredient contents shown in Table 3 below, irbesartan, β-carotene, microcrystalline cellulose, pregelatinized starch, lactose, and croscarmellose sodium were sieved through a No. 35 sieve and mixed in a high-speed mixer for 5 minutes to prepare a mixture. Meanwhile, Poloxamer 188 was dissolved in purified water (100 mg/tablet) to prepare a binding solution, followed by kneading, granulation and drying. The dried material was placed in a fluidized bed coater. Meanwhile, cellulose acetate (acetyl group 32.0%) and cellulose acetate (acetyl group 39.8%) were dissolved in a (2:8) mixed solvent of ethanol and methylene chloride (800 mg/tablet) to prepare a solution which was then coated on the granules in a fluidized bed coater (GPCG-1: Glatt, Germany). After completion of the coating process, drying was carried out to prepare angiotensin-II-receptor blocker delayed-release granules.

### 2) Compression

The delayed-release granules prepared in Section 1), and colloidal silicon dioxide and magnesium stearate at the contents shown in Table 3 below were placed in a double cone mixer, followed by final mixing for 4 minutes. The final mixture was compressed into tablets using a rotary tablet press (MRC-33: Sejong, South Korea), thereby preparing uncoated tablets containing β-carotene and irbesartan.

### <Example 20> Preparation of uncoated tablets containing a hepatitis-preventing and inhibiting agent and an angiotensin-II-receptor blocker

### 1) Preparation of delayed-release granules

According to the ingredient contents shown in Table 3 below, valsartan, silymarin, microcrystalline cellulose, and crosslinked polyvinylpyrrolidone were sieved through a No. 35 sieve and mixed in a high-speed mixer for 5 minutes to prepare a mixture. Meanwhile, polyvinylpyrrolidone was dissolved in purified water (70 mg/tablet) to prepare a binding solution, followed by kneading, granulation and drying. The dried material was placed in a fluidized bed coater. Meanwhile, a solution of hypromellose in purified water (70 mg/tablet) and a solution of hypromellose phthalate (HP-55) in a (8:2) mixed solvent of ethanol and purified water (120 mg/tablet) were coated on the granules in a fluidized bed coater (GPCG-1: Glatt, Germany). After completion of the coating process, drying was carried out to prepare delayed-release granules.

### 2) Compression

The delayed-release granules prepared in Section 1) and magnesium stearate at the content shown in Table 3 below were placed in a double cone mixer, followed by final mixing for 4 minutes. The final mixture was compressed into tablets using a rotary tablet press (MRC-33: Sejong, South Korea), thereby preparing uncoated tablets containing silymarin and valsartan.

### <Example 21> Preparation of uncoated tablets containing a hepatitis-preventing and inhibiting agent and an angiotensin-II-receptor blocker

### 1) Preparation of delayed-release granules

According to the ingredient contents shown in Table 3 below, losartan potassium, biphenyldimethyldicarboxylate, microcrystalline cellulose, sodium starch glycolate, and lactose were sieved through a No. 35 sieve and mixed in a high-speed mixer for 5 minutes to prepare a mixture. Meanwhile, hydroxypropylcellulose was dissolved in purified water (40 mg/tablet) to prepare a binding solution, followed by kneading, granulation and drying. The dried material was placed in a fluidized bed coater. Meanwhile, hypromellose phthalate (HP-55) and polyethylene glycol 6000 were dissolved in a (8:2) mixed solvent of ethanol and purified water (120 mg/tablet) to prepare a solution which was then coated on the granules in a fluidized bed coater (GPCG-1: Glatt, Germany). After completion of the coating process, drying was carried out. The dried material and colloidal silicon dioxide were placed in a double cone mixer, followed by mixing to prepare delayed-release granules containing biphenyldimethyldicarboxylate and losartan.

### 2) Compression

The delayed-release granules prepared in Section 1) and magnesium stearate at the content shown in Table 3 below were placed, followed by final mixing for 4 minutes. The final mixture was compressed into tablets using a rotary tablet press (MRC-33: Sejong, South Korea), thereby preparing delayed-release uncoated tablets.

### <Example 22> Preparation of film-coated tablets containing angiotensin-II-receptor blocker delayed-release granules and a hepatitis-preventing and inhibiting agent prior-release film

### 1) Preparation of losartan potassium-containing delayed-release uncoated tablets

Delayed-release uncoated tablets were prepared in the same manner as in Sections 1) and 2) of Example 21 and according to the ingredient compositions and contents shown in Table 3 below, except that biphenyldimethyldicarboxylate was not incorporated.

### 2) Acetylcysteine-containing film coating

The uncoated tablets prepared in Section 1) were placed in a Hi-coater (SFC-30N, Sejong Machinery Co., Ltd., South Korea). According to the ingredient compositions shown in Table 3 below, acetylcysteine, hypromellose 2910, hydroxypropylcellulose, titanium oxide, and talc were dissolved and dispersed in purified water (260 mg/tablet) to prepare a coating solution which was then coated thereon to prepare film-coated tablets including an acetylcysteine-containing prior-release film layer and losartan potassium-containing delayed-release granules.

### <Example 23> Preparation of film-coated tablets containing angiotensin-II-receptor blocker delayed-release granules and a hepatitis-preventing and inhibiting agent prior-release film

### 1) Preparation of delayed-release uncoated tablets

According to the ingredient contents shown in Table 3 below, eprosartan, selenized yeast, microcrystalline cellulose, sodium starch glycolate, and lactose were sieved through a No. 35 sieve and mixed in a high-speed mixer for 5 minutes to prepare a mixture. Meanwhile, hydroxypropylcellulose was dissolved in purified water (40 mg/tablet) to prepare a binding solution, followed by kneading, granulation and drying. The dried material was placed in a fluidized bed coater. Meanwhile, hypromellose phthalate (HP-55) and polyethylene glycol 6000 were dissolved in ethanol (220 mg/tablet) and methylene chloride (980 mg/tablet) to prepare a solution which was then coated on the granules in a fluidized bed coater (GPCG-1: Glatt, Germany). After completion of the coating process, drying was carried out. The dried material and colloidal silicon dioxide were placed in a double cone mixer, followed by mixing to prepare delayed-release granules.

### 2) Compression

The delayed-release granules prepared in Section 1) and magnesium stearate at the content shown in Table 3 below were placed in a double cone mixer, followed by final mixing for 4 minutes. The final mixture was compressed into tablets using a rotary tablet press (MRC-33: Sejong, South Korea), thereby preparing uncoated tablets containing selenized yeast and eprosartan.

### 3) ATSO (Coriolus versicolor polysaccharide)-containing film coating

The uncoated tablets prepared in Section 1) were placed in a Hi-coater (SFC-30N, Sejong Machinery Co., Ltd., South Korea). According to the ingredient compositions shown in Table 3 below, ATSO (Coriolus versicolor polysaccharide), hypromellose 2910, hydroxypropylcellulose, titanium oxide, and talc were dissolved and dispersed in purified water (260 mg/tablet) to prepare a coating solution which was then coated thereon to prepare film-coated tablets including a prior-release film layer containing Coriolus versicolor polysaccharide (ATSO) and delayed-release granules containing selenized yeast and losartan.

### <Example 24> Preparation of film-coated tablets containing anitiotensin-II-receptor blocker granules and a hepatitis-preventing and inhibiting agent prior-release film

### 1) Preparation of telmisartan-containing delayed-release uncoated tablets

Delayed-release uncoated tablets were prepared in the same manner as in Sections 1) and 2) of Example 1 and according to the ingredient compositions and contents shown in Table 3 below.

### 2) Citiolone-containing film coating

The uncoated tablets prepared in Section 1) were placed in a Hi-coater (SFC-30N, Sejong Machinery Co., Ltd., South Korea). According to the ingredient compositions shown in Table 3 below, citiolone, hypromellose 2910, hydroxypropylcellulose, titanium oxide, and talc were dissolved and dispersed in purified water (260 mg/tablet) to prepare a coating solution which was then coated thereon to prepare film-coated tablets including a citiolone-containing prior-release film layer and telmisartan-containing delayed-release granules.

### <Example 25> Preparation of delayed-release film-coated tablets containing a hepatitis-preventing and inhibiting agent and an angiotensin-II-receptor blocker

### 1) Preparation of uncoated tablets

According to the ingredient compositions shown in Table 4 below, losartan potassium, urazamide, microcrystalline cellulose, pregelatinized starch, a polyvinylpyrrolidone copolymer, and colloidal silicon dioxide were sieved through a No. 35 sieve and mixed in a double cone mixer for 30 minutes to prepare a mixture. To the mixture was added magnesium stearate, followed by mixing for 4 minutes. The final mixture was compressed into tablets using a rotary tablet press (MRC-33: Sejong, South Korea), thereby preparing uncoated tablets containing urazamide and losartan potassium.

### 2) Delayed-release film coating

The uncoated tablets prepared in Section 1) were placed in a Hi-coater (SFC-30N, Sejong Machinery Co., Ltd., South Korea). Meanwhile, ethylcellulose and Acryl-EZE were dissolved and dispersed in purified water (400 mg/tablet) to prepare a coating solution which was then coated thereon to prepare delayed-release film-coated tablets having a delayed-release film and containing urazamide and losartan potassium.

### <Example 26> Preparation of delayed-release film-coated tablets containing a hepatitis-preventing and inhibiting agent and an angiotensin-II-receptor blocker

### 1) Preparation of uncoated tablets

Except that fraction flavonoid purifiee micronise was contained in place of urazamide, uncoated tablets were prepared in the same manner as in Section 1) of Example 25 and according to the ingredient compositions and contents shown in Table 4 below.

### 2) Delayed-release film coating

Delayed-release film-coated tablets containing fraction flavonoid purifiee micronise and losartan potassium were prepared in the same manner as in Section 2) of Example 25 and according to the ingredient compositions and contents shown in Table 4 below.

### <Example 27> Preparation of delayed-release film-coated tablets containing a hepatitis-preventing and inhibiting agent and an angiotensin-II-receptor blocker

### 1) Preparation of uncoated tablets

Except that cicloxilic acid was contained in place of urazamide, uncoated tablets were prepared in the same manner as in Section 1) of Example 25 and according to the ingredient compositions and contents shown in Table 4 below.

### 2) Film coating containing a delayed-release film

Delayed-release film-coated tablets containing cicloxilic acid and losartan potassium were prepared in the same manner as in Section 2) of Example 25 and according to the ingredient compositions and contents shown in Table 4 below.

### <Example 28> Preparation of film-coated tablets containing a hepatitis-preventing and inhibiting agent and an angiotensin-II-receptor blocker

### 1) Preparation of uncoated tablets

Except that riboflavin tetrabutyrate, ascorbic acid, and pyridoxamine hydrochloride were contained in place of urazamide, uncoated tablets were prepared in the same manner as in Section 1) of Example 25 and according to the ingredient compositions and contents shown in Table 4 below.

### 2) Delayed-release film coating

Delayed-release film-coated tablets containing riboflavin tetrabutyrate, ascorbic acid, pyridoxamine hydrochloride and losartan potassium were prepared in the same manner as in Section 2) of Example 25 and according to the ingredient compositions and contents shown in Table 4 below.

### <Example 29> Preparation of film-coated tablets containing a hepatitis-preventing and inhibiting agent and an angiotensin-II-receptor blocker

### 1) Preparation of uncoated tablets

Except that agaro-oligosaccharide and melatonin were contained in place of urazamide, uncoated tablets were prepared in the same manner as in Section 1) of Example 25 and according to the ingredient compositions and contents shown in Table 4 below.

### 2) Film coating containing a delayed-release film

The uncoated tablets prepared in Section 1) were placed in a Hi-coater (SFC-30N, Sejong Machinery Co., Ltd., South Korea). Meanwhile, polyvinylpyrrolidone, ethylcellulose and Acryl-EZE were dissolved and dispersed in purified water (400 mg/tablet) to prepare a coating solution which was then coated thereon to prepare delayed-release film-coated tablets having a delayed-release film and containing agaro-oligosaccharide, melatonin and losartan potassium.

### <Example 30> Preparation of delayed-release film-coated tablets containing a hepatitis-preventing and inhibiting agent and an angiotensin-II-receptor blocker

### 1) Preparation of uncoated tablets

Except that ursodeoxycholic acid was contained in place of urazamide, uncoated tablets were prepared in the same manner as in Section 1) of Example 25 and according to the ingredient compositions and contents shown in Table 4 below.

### 2) Film coating containing a delayed-release film

Delayed-release film-coated tablets containing ursodeoxycholic acid and losartan potassium were prepared in the same manner as in Section 2) of Example 29 and according to the ingredient compositions and contents shown in Table 4 below.

### <Example 31> Preparation of delayed-release film-coated tablets containing a hepatitis-preventing and inhibiting agent and an angiotensin-II-receptor blocker

### 1) Preparation of uncoated tablets

Except that naphthyl acetic acid and azintamide were contained in place of urazamide, uncoated tablets were prepared in the same manner as in Section 1) of Example 25 and according to the ingredient compositions and contents shown in Table 4 below.

### 2) Film coating containing a delayed-release film

Delayed-release film-coated tablets containing naphthyl acetic acid, azintamide and losartan potassium were prepared in the same manner as in Section 2) of Example 29 and according to the ingredient compositions and contents shown in Table 4 below.

### <Example 32> Preparation of multi-layered tablets containing a hepatitis-preventing and inhibiting agent prior-release layer and an angiotensin-II-receptor blocker delayed-release layer

### 1) Preparation of valsartan delayed-release layer

According to the ingredient contents shown in Table 5 below, valsartan, microcrystalline cellulose, and crosslinked polyvinylpyrrolidone were sieved through a No. 35 sieve and mixed in a high-speed mixer for 5 minutes to prepare a mixture. Meanwhile, polyvinylpyrrolidone was dissolved in purified water (80 mg/tablet) to prepare a binding solution, followed by kneading, granulation and drying. The dried material was placed in a fluidized bed coater. Meanwhile, hypromellose was dissolved in purified water (32 mg/tablet) to prepare a solution which was then coated on the granules in a fluidized bed coater (GPCG-1: Glatt, Germany) (first coating). Meanwhile, cellulose acetate (acetyl group 32%) and cellulose acetate (acetyl group 39.8%) were dissolved in a (8:2) mixed solvent of ethanol and purified water (640 mg/tablet) to prepare a solution which was then coated on the above-coated material in a fluidized bed coater (GPCG-1: Glatt, Germany) (second coating). After completion of the coating process, drying was carried out to prepare a valsartan delayed-release layer.

### 2) Preparation of hepatitis-preventing and inhibiting agent prior-release layer

According to the ingredient compositions and contents shown in Table 5 below, ubidecarenone, haematoporphyrin, microcrystalline cellulose, pregelatinized starch, and corn starch were sieved through a No. 35 sieve and mixed in a high-speed mixer. Meanwhile, hydroxypropylcellulose was dissolved in purified water (60 mg/tablet) to prepare a binding solution. The binding solution and the mixture of main ingredients were placed in a high-speed mixer, followed by kneading. After completion of the kneading process, the kneaded material was granulated using an oscillator with a No. 18 sieve, and the granules were dried in a hot-water dryer at 30°C. After completion of the drying process, the granules were sieved again through an F-type grinder equipped with a No. 20 sieve to prepare a hepatitis-preventing and inhibiting agent prior-release layer.

### 3) Compression

The semi-finished product of Section 1) and magnesium stearate were mixed and placed in a first powder feeder. The semi-finished product of Section 2) and magnesium stearate were mixed and placed in a second powder feeder. Using a multiple tablet press (MRC-37T: Sejong, South Korea), the products in the feeders were compressed into tablets under such conditions that the interlayer incorporation can be minimized, thereby preparing multi-layered tablets.

### <Example 33> Preparation of multi-layered tablets containing a hepatitis-preventing and inhibiting agent prior-release layer and an angiotensin-II-receptor blocker delayed-release layer

### 1) Preparation of losartan delayed-release layer

According to the ingredient contents shown in Table 5 below, losartan potassium, microcrystalline cellulose, sodium starch glycolate, and lactose were sieved through a No. 35 sieve and mixed in a high-speed mixer for 5 minutes to prepare a mixture. Meanwhile, hydroxypropylcellulose was dissolved in purified water (40 mg/tablet) to prepare a binding solution, followed by kneading, granulation and drying. The dried material was placed in a fluidized bed coater. Meanwhile, hypromellose, cellulose acetate (acetyl group 32%) and cellulose acetate (acetyl group 39.8%) were dissolved in a (2:8) mixed solvent of ethanol and methylene chloride (820 mg/tablet) to prepare a solution which was then coated on the granules in a fluidized bed coater (GPCG-1: Glatt, Germany). After completion of the coating process, drying was carried out to prepare a losartan delayed-release layer.

### 2) Preparation of hepatitis-preventing and inhibiting agent prior-release layer

According to the ingredient compositions and contents shown in Table 5 below, arginine hydrochloride, ornithine hydrochloride, microcrystalline cellulose, pregelatinized starch, and corn starch were sieved through a No. 35 sieve and mixed in a high-speed mixer. Meanwhile, hydroxypropylcellulose was dissolved in purified water (80 mg/tablet) to prepare a binding solution. The binding solution and the mixture of main ingredients were placed in a high-speed mixer, followed by kneading. After completion of the kneading process, the kneaded material was granulated using an oscillator with a No. 18 sieve, and the granules were dried in a hot-water dryer at 30°C. After completion of the drying process, the granules were sieved again through an F-type grinder equipped with a No. 20 sieve to prepare a hepatitis-preventing and inhibiting agent prior-release layer.

### 3) Compression and coating

The semi-finished product of Section 1) and magnesium stearate were mixed and placed in a first powder feeder. The semi-finished product of Section 2) and magnesium stearate were mixed and placed in a second powder feeder. Using a multiple tablet press (MRC-37T: Sejong, South Korea), the products in the feeders were compressed into tablets under such conditions that the interlayer incorporation can be minimized, thereby preparing multi-layered tablets.

### <Example 34> Preparation of multi-layered tablets containing a hepatitis-preventing and inhibiting agent prior-release layer and an angiotensin-II-receptor blocker delayed-release layer

### 1) Preparation of losartan delayed-release layer

According to the ingredient contents shown in Table 5 below, losartan potassium, microcrystalline cellulose, sodium starch glycolate, and lactose were sieved through a No. 35 sieve and mixed in a high-speed mixer for 5 minutes to prepare a mixture. Meanwhile, hydroxypropylcellulose was dissolved in purified water (40 mg/tablet) to prepare a binding solution, followed by kneading, granulation and drying. The dried material was placed in a fluidized bed coater. Meanwhile, hypromellose phthalate (HP-50) and polyethylene glycol 6000 were dissolved in a (8:2) mixed solvent of ethanol and purified water (600 mg/tablet) to prepare a solution which was then coated on the granules in a fluidized bed coater (GPCG-1: Glatt, Germany). After completion of the coating process, drying was carried out to prepare a losartan delayed-release layer.

### 2) Preparation of hepatitis-preventing and inhibiting agent prior-release layer

According to the ingredient compositions and contents shown in Table 5 below, erdosteine, guaiacol, creatine, microcrystalline cellulose, pregelatinized starch, and corn starch were sieved through a No. 35 sieve and mixed in a high-speed mixer. Meanwhile, hydroxypropylcellulose was dissolved in purified water (80 mg/tablet) to prepare a binding solution. The binding solution and the mixture of main ingredients were placed in a high-speed mixer, followed by kneading. After completion of the kneading process, the kneaded material was granulated using an oscillator with a No. 18 sieve, and the granules were dried in a hot-water dryer at 30°C. After completion of the drying process, the granules were sieved again through an F-type grinder equipped with a No. 20 sieve to prepare hepatitis-preventing and inhibiting agent prior-release layer.

### 3) Compression and coating

The semi-finished product of Section 1) and magnesium stearate were mixed and placed in a first powder feeder. The semi-finished product of Section 2) and magnesium stearate were mixed and placed in a second powder feeder. Using a multiple tablet press (MRC-37T: Sejong, South Korea), the products in the feeders were compressed into tablets under such conditions that the interlayer incorporation can be minimized. Meanwhile, hypromellose 2910, hydroxypropylcellulose, titanium oxide, and talc were dissolved and dispersed in a (8:2) mixed solvent of ethanol and purified water (600 mg/tablet) to prepare a coating solution. The compressed tablets were coated with the coating solution in a Hi-coater (SFC-30N: Sejong Machinery Co., Ltd., South Korea) to form a film-coated layer, thereby preparing multi-layered tablets.

### <Example 35> Preparation of press-coated tablets containing a hepatitis-preventing and inhibiting agent prior-release layer and an angiotensin-II-receptor blocker delayed-release layer

### 1) Preparation of candesartan cilexetil delayed-release inner core tablets

According to the ingredient compositions and contents shown in Table 5 below, candesartan cilexetil, lactose, corn starch, polyethylene glycol 6000 and carboxymethylcellulose calcium were sieved through a No. 35 sieve and mixed in a high-speed mixer. Meanwhile, hydroxypropylcellulose was dissolved in purified water (300 mg/tablet) to prepare a binding solution. The binding solution and the mixture of main ingredients were placed in a high-speed mixer, followed by kneading. After completion of the kneading process, the kneaded material was granulated using an oscillator with a No. 18 sieve, and the granules were dried in a hot-water dryer at 30°C. After completion of the drying process, the granules were sieved again through an F-type grinder equipped with a No. 20 sieve. The sieved material was placed in a double cone mixer, and magnesium stearate was added thereto, followed by final mixing. The final mixture was compressed into tablets using a rotary tablet press (MRC-33: Sejong, South Korea). The compressed tablets were placed in a Hi-coater (SFC-30N, Sejong Machinery Co., Ltd., South Korea) and then coated with a coating solution of hypromellose acetate succinate in a (8:2) mixed solvent of ethanol and purified water (550 mg/tablet), thereby preparing candesartan cilexetil delayed-release coated inner core tablets.

### 2) Preparation of hepatitis-preventing and inhibiting agent prior-release outer layer granules

According to the ingredient compositions and contents shown in Table 4 below, riboflavin, cholecalciferol, microcrystalline cellulose, pregelatinized starch, and corn starch were sieved through a No. 35 sieve and mixed in a high-speed mixer. Meanwhile, hydroxypropylcellulose was dissolved in purified water (80 mg/tablet) to prepare a binding solution. The binding solution and the mixture of main ingredients were placed in a high-speed mixer, followed by kneading. After completion of the kneading process, the kneaded material was granulated using an oscillator with a No. 18 sieve, and the granules were dried in a hot-water dryer at 30°C. After completion of the drying process, the granules were sieved again through an F-type grinder equipped with a No. 20 sieve. The sieved material was placed in a double cone mixer, and magnesium stearate was added thereto, followed by final mixing to prepare hepatitis-preventing and inhibiting agent prior-release outer layer granules.

### 3) Compression

Using a press-coated tablet press (RUD-1: Kilian), press-coated tablets were prepared such that the candesartan cilexetil delayed-release uncoated tablets prepared in Section 1) were placed inside the tablet, and the hepatitis-preventing and inhibiting agent prior-release granules prepared in Section 2) were placed outside the tablet.

### <Example 36> Preparation of press-coated tablets containing a hepatitis-preventing and inhibiting agent prior-release layer and an angiotensin-II-receptor blocker delayed-release layer

### 1) Preparation of eprosartan delayed-release inner core tablets

According to the ingredient contents shown in Table 5 below, eprosartan, microcrystalline cellulose, sodium starch glycolate, and lactose were sieved through a No. 35 sieve and mixed in a high-speed mixer for 5 minutes to prepare a mixture. Meanwhile, hydroxypropylcellulose was dissolved in purified water (40 mg/tablet) to prepare a binding solution, followed by kneading, granulation and drying. After completion of the drying process, the granules were sieved again through an F-type grinder equipped with a No. 20 sieve. The sieved material was placed in a double cone mixer, and magnesium stearate was added thereto, followed by final mixing. The final mixture was compressed into tablets using a rotary tablet press (MRC-33: Sejong, South Korea). The compressed tablets were placed in a Hi-coater (SFC-30N, Sejong Machinery Co., Ltd., South Korea) and then coated with a coating solution of hypromellose phthalate (HP-50) and polyvinyl acetate in a (8:2) mixed solvent of ethanol and purified water (200 mg/tablet), thereby preparing eprosartan delayed-release coated inner core tablets.

### 2) Preparation of hepatitis-preventing and inhibiting agent prior-release outer layer granules

According to the ingredient compositions and contents shown in Table 5 below, L-glutathione, L-glutamine, microcrystalline cellulose, pregelatinized starch, and corn starch were sieved through a No. 35 sieve and mixed in a high-speed mixer. Meanwhile, hydroxypropylcellulose was dissolved in purified water (80 mg/tablet) to prepare a binding solution. The binding solution and the mixture of main ingredients were placed in a high-speed mixer, followed by kneading. After completion of the kneading process, the kneaded material was granulated using an oscillator with a No. 18 sieve, and the granules were dried in a hot-water dryer at 30°C. After completion of the drying process, the granules were sieved again through an F-type grinder equipped with a No. 20 sieve. The sieved material was placed in a double cone mixer, and magnesium stearate was added thereto, followed by final mixing to prepare hepatitis-preventing and inhibiting agent prior-release outer layer granules.

### 3) Compression and coating

Using a press-coated tablet press (RUD-1: Kilian), press-coated tablets were prepared such that the eprosartan delayed-release uncoated tablets prepared in Section 1) were placed inside the tablet, and the hepatitis-preventing and inhibiting agent prior-release granules prepared in Section 2) were placed outside the tablet. Meanwhile, hypromellose 2910, titanium oxide, and talc were dissolved and dispersed in a (8:2) mixed solvent of ethanol and purified water (800 mg/tablet) to prepare a coating solution. The press-coated tablets were placed in a Hi-coater (SFC-30N, Sejong Machinery Co., Ltd., South Korea) and then coated with the coating solution to prepare coated press-coated tablets.

### <Example 37> Preparation of press-coated tablets containing a hepatitis-preventing and inhibiting agent prior-release layer and an angiotensin-11-receptor blocker delayed-release layer

### 1) Preparation of losartan delayed-release inner core tablets

Uncoated tablets were compressed in the same manner as in Section 1) of Example 8 and according to the ingredient compositions and contents shown in Table 5 below. The compressed tablets were placed in a Hi-coater (SFC-30N, Sejong Machinery Co., Ltd., South Korea). A solution of hypromellose in purified water (10 mg/tablet) was coated thereon (first coating). Then, a coating solution of Acryl-EZE in purified water (80 mg/tablet) was coated again on the granules (second coating), thereby preparing eprosartan delayed-release coated inner core tablets.

### 2) Preparation of hepatitis-preventing and inhibiting agent prior-release outer layer granules

According to the ingredient compositions and contents shown in Table 5 below, sodium selenite, a ginko biloba extract, microcrystalline cellulose, pregelatinized starch, and corn starch were sieved through a No. 35 sieve and mixed in a high-speed mixer. Meanwhile, hydroxypropylcellulose was dissolved in purified water (80 mg/tablet) to prepare a binding solution. The binding solution and the mixture of main ingredients were placed in a high-speed mixer, followed by kneading. After completion of the kneading process, the kneaded material was granulated using an oscillator with a No. 18 sieve, and the granules were dried in a hot-water dryer at 30°C. After completion of the drying process, the granules were sieved again through an F-type grinder equipped with a No. 20 sieve. The sieved material was placed in a double cone mixer, and magnesium stearate was added thereto, followed by final mixing to prepare hepatitis-preventing and inhibiting agent prior-release outer layer granules.

### 3) Compression and coating

Using a press-coated tablet press (RUD-1: Kilian), press-coated tablets were prepared such that the losartan delayed-release uncoated tablets prepared in Section 1) were placed inside the tablet, and the hepatitis-preventing and inhibiting agent prior-release granules prepared in Section 2) were placed outside the tablet.

### <Example 38> Preparation of press-coated tablets containing a hepatitis-preventing and inhibiting agent prior-release layer and an angiotensin-II-receptor blocker delayed-release layer

### 1) Preparation of telmisartan delayed-release inner core tablets

According to the ingredient contents shown in Table 5 below, telmisartan, microcrystalline cellulose, sorbitol, and meglumine were sieved through a No. 35 sieve and mixed in a high-speed mixer for 5 minutes to prepare a mixture. Meanwhile, polyvinylpyrrolidone and sodium hydroxide were dissolved in purified water (70 mg/tablet) to prepare a binding solution, followed by kneading, granulation and drying. After completion of the drying process, the granules were sieved again through an F-type grinder equipped with a No. 20 sieve. The sieved material was placed in a double cone mixer, and magnesium stearate was added thereto, followed by final mixing. The final mixture was compressed into tablets using a rotary tablet press (MRC-33: Sejong, South Korea). The compressed tablets were placed in a Hi-coater (SFC-30N, Sejong Machinery Co., Ltd., South Korea). Meanwhile, hypromellose phthalate (HP-55) and polyethylene glycol 6000 were dissolved in a (8:2) mixed solvent of ethanol and purified water (80 mg/tablet) to prepare a coating solution which was then coated thereon to prepare telmisartan delayed-release coated inner core tablets.

### 2) Preparation of hepatitis-preventing and inhibiting agent prior-release outer layer granules

According to the ingredient compositions and contents shown in Table 5 below, butylated hydroxyanisole, ornithine, microcrystalline cellulose, pregelatinized starch, and corn starch were sieved through a No. 35 sieve and mixed in a high-speed mixer. Meanwhile, hydroxypropylcellulose was dissolved in purified water (80 mg/tablet) to prepare a binding solution. The binding solution and the mixture of main ingredients were placed in a high-speed mixer, followed by kneading. After completion of the kneading process, the kneaded material was granulated using an oscillator with a No. 18 sieve, and the granules were dried in a hot-water dryer at 30°C. After completion of the drying process, the granules were sieved again through an F-type grinder equipped with a No. 20 sieve. The sieved material was placed in a double cone mixer, and magnesium stearate was added thereto, followed by final mixing to prepare hepatitis-preventing and inhibiting agent prior-release outer layer granules.

### 3) Compression and coating

Using a press-coated tablet press (RUD-1: Kilian), press-coated tablets were prepared such that the telmisartan delayed-release uncoated tablets prepared in Section 1) were placed inside the tablet, and the hepatitis-preventing and inhibiting agent prior-release granules prepared in Section 2) were placed outside the tablet. Meanwhile, hypromellose 2910, titanium oxide, and talc were dissolved and dispersed in a (8:2) mixed solvent of ethanol and purified water (350 mg/tablet) to prepare a coating solution. The press-coated tablets were placed in a Hi-coater (SFC-30N, Sejong Machinery Co., Ltd., South Korea) and then coated with the coating solution to prepare coated press-coated tablets.

### <Example 39> Preparation of osmotic press-coated tablets

### 1) Preparation of candesartan cilexetil delayed-release osmotic inner core tablets

According to the ingredient compositions and contents shown in Table 6 below, candesartan cilexetil, microcrystalline cellulose, and sodium chloride were sieved through a No. 35 sieve and mixed in a double cone mixer for 30 minutes to prepare a mixture. To the mixture was added magnesium stearate, followed by mixing for 4 minutes. The final mixture was compressed into tablets using a rotary tablet press (MRC-33: Sejong, South Korea). After completion of the compression process, ethylcellulose was dispersed in purified water (50 mg/tablet) to prepare a dispersion which was then coated on the inner core tablets in a Hi-coater (SFC-30N, Sejong Machinery Co., Ltd., South Korea), thereby preparing osmotic inner core tablets.

### 2) Preparation of hepatitis-preventing and inhibiting agent prior-release outer layer granules

According to the ingredient compositions and contents shown in Table 6 below, arginine sodium, microcrystalline cellulose, pregelatinized starch, and corn starch were sieved through a No. 35 sieve and mixed in a high-speed mixer. Meanwhile, hydroxypropylcellulose was dissolved in purified water (80 mg/tablet) to prepare a binding solution. The binding solution and the mixture of main ingredients were placed in a high-speed mixer, followed by kneading. After completion of the kneading process, the kneaded material was granulated using an oscillator with a No. 18 sieve, and the granules were dried in a hot-water dryer at 30°C. After completion of the drying process, the granules were sieved again through an F-type grinder equipped with a No. 20 sieve. The sieved material was placed in a double cone mixer, and magnesium stearate was added thereto, followed by final mixing to prepare hepatitis-preventing and inhibiting agent prior-release outer layer granules.

### 3) Compression and coating

Using a press-coated tablet press (RUD-1: Kilian), press-coated tablets were prepared such that the candesartan cilexetil delayed-release osmotic inner core tablets prepared in Section 1) were placed as inner core tablets, and the hepatitis-preventing and inhibiting agent prior-release outer layer prepared in Section 2) was placed outside the tablet. Meanwhile, hypromellose 2910, hydroxypropylcellulose, titanium oxide, and talc were dissolved and dispersed in 80% ethanol to prepare a coating solution. The press-coated tablets were placed in a Hi-coater (SFC-30N, Sejong Machinery Co., Ltd., South Korea) and then coated with the coating solution to prepare coated osmotic press-coated tablets.

### <Example 40> Preparation of capsules (granules-granules)

### 1) Preparation of olmesartan delayed-release granules

According to the ingredient compositions and contents shown in Table 6 below, olmesartan medoxomil, microcrystalline cellulose, and pregelatinized starch were sieved through a No. 35 sieve and mixed in a high-speed mixer for 5 minutes to prepare a mixture. Meanwhile, hydroxypropylcellulose was dissolved in purified water (40 mg/tablet) to prepare a binding solution, followed by kneading, granulation and drying. The dried material was placed in a fluidized bed coater. Meanwhile, polyvinyl acetate was dissolved in a (2:8) mixed solvent of ethanol and methylene chloride (240 mg/tablet) to prepare a solution which was then coated on the granules in a fluidized bed coater (GPCG-1: Glatt, Germany). After completion of the coating process, drying was carried out to prepare olmesartan delayed-release granules.

### 2) Preparation of hepatitis-preventing and inhibiting agent prior-release granules

According to the ingredient compositions and contents shown in Table 6 below, protoporphyrin disodium, microcrystalline cellulose, pregelatinized starch, and corn starch were sieved through a No. 35 sieve and mixed in a high-speed mixer. Meanwhile, hydroxypropylcellulose was dissolved in purified water (80 mg/tablet) to prepare a binding solution. The binding solution and the mixture of main ingredients were placed in a high-speed mixer, followed by kneading. After completion of the kneading process, the kneaded material was granulated using an oscillator with a No. 18 sieve, and the granules were dried in a hot-water dryer at 30°C. After completion of the drying process, the granules were sieved again through an F-type grinder equipped with a No. 20 sieve to prepare hepatitis-preventing and inhibiting agent prior-release granules.

### 3) Mixing and capsule filling

According to the ingredient compositions and contents shown in Table 6 below, the final compositions of Sections 1) and 2) were mixed in a double cone mixer. To the mixture was added magnesium stearate, followed by final mixing in a double cone mixer. The final mixture was placed in a powder feeder and then filled in No. 1 gelatin hard capsules using a capsule filling machine to prepare a formulation in the form of a capsule.

### <Example 41> Preparation of capsules (granules-tablets)

### 1) Preparation of valsartan delayed-release granules

According to the ingredient contents shown in Table 6 below, valsartan, microcrystalline cellulose, and crosslinked polyvinylpyrrolidone were sieved through a No. 35 sieve and mixed in a high-speed mixer for 5 minutes to prepare a mixture. Meanwhile, polyvinylpyrrolidone was dissolved in purified water (70 mg/tablet) to prepare a binding solution, followed by kneading, granulation and drying. The dried material was placed in a fluidized bed coater. Meanwhile, a solution of hypromellose in purified water (70 mg/tablet) and a solution of hypromellose phthalate (HP-55) in a (8:2) mixed solvent of ethanol and purified water (120 mg/tablet) were prepared and then coated on the granules in a fluidized bed coater (GPCG-1: Glatt, Germany). After completion of the coating process, drying was carried out to prepare valsartan delayed-release granules.

### 2) Preparation of hepatitis-preventing and inhibiting agent prior-release tablets

According to the ingredient compositions and contents shown in Table 6 below, hymecromone, microcrystalline cellulose, pregelatinized starch, and corn starch were sieved through a No. 35 sieve and mixed in a high-speed mixer. Meanwhile, hydroxypropylcellulose was dissolved in purified water (80 mg/tablet) to prepare a binding solution. The binding solution and the mixture of main ingredients were placed in a high-speed mixer, followed by kneading. After completion of the kneading process, the kneaded material was granulated using an oscillator with a No. 18 sieve, and the granules were dried in a hot-water dryer at 30°C. After completion of the drying process, the granules were sieved again through an F-type grinder equipped with a No. 20 sieve. The sieved material and magnesium stearate were placed in a double cone mixer, followed by final mixing. The final mixture was compressed into tablets using a rotary tablet press (MRC-33, Sejong Machinery Co., Ltd., South Korea), thereby preparing hepatitis-preventing and inhibiting agent prior-release tablets.

### 3) Mixing and capsule filling

The final products of Sections 1) and 2) were filled in capsules using a capsule filling machine, thereby preparing a formulation in the form of a capsule.

### <Example 42> Preparation of capsules (pellets-pellets)

### 1) Preparation of losartan delayed-release pellets,

According to the ingredient compositions and contents shown in Table 6 below, sugar seeds (sugar sphere) were sieved through a No. 35 sieve and placed in a fluidized bed granulator (GPCG 1: Glatt). Meanwhile, hypromellose and losartan potassium were dissolved in a (8:2) mixed solution of ethanol and purified water (500 mg/tablet) to prepare a binding solution which was then sprayed thereon to form pellets, followed by drying. Then, a solution of hypromellose phthalate (HP-55) in a (2:8) mixed solvent of ethanol and methylene chloride (1200 mg/tablet) was sprayed on the granules, thereby preparing losartan delayed-release pellets.

### 2) Preparation of hepatitis-preventing and inhibiting agent prior-release pellets

According to the ingredient compositions and contents shown in Table 6 below, sugar seeds (sugar sphere) were sieved through a No. 35 sieve and placed in a fluidized bed granulator (GPCG 1: Glatt). Meanwhile, hydroxypropylcellulose, haematoporphyrin, and a ginko biloba extract were dissolved in a (8:2) mixed solution of ethanol and purified water (150 mg/tablet) to prepare a binding solution which was then sprayed thereon to form pellets, followed by drying to prepare hepatitis-preventing and inhibiting agent prior-release pellets.

### 3) Mixing and capsule filling

The final compositions of Sections 1) and 2) were filled in No. 1 gelatin hard capsules using a capsule filling machine, thereby preparing a formulation in the form of a capsule.

### <Example 43> Preparation of capsules (pellets-tablets)

### 1) Preparation of eprosartan delayed-release pellets

Eprosartan delayed-release pellets were prepared in the same manner as in Section 1) of Example 42 and according to the ingredient compositions and contents shown in Example 43 of Table 6, except that eprosartan was used in place of losartan potassium.

### 2) Preparation of hepatitis-preventing and inhibiting agent prior-release tablets

Hepatitis-preventing and inhibiting agent prior-release pellets were prepared in the same manner as in Section 2) of Example 41 and according to the ingredient compositions and contents given in Example 43 of Table 6, except that guaiacol, cholecalciferol and arginine sodium were used in place of hymecromone.

### 3) Mixing and capsule filling

The final products of Sections 1) and 2) were filled in capsules using a capsule filling machine, thereby preparing a formulation in the form of a capsule.

### <Example 44> Preparation of capsules (tablets-granules)

### 1) Preparation of losartan delayed-release tablets

Uncoated tablets were prepared in the same manner as in Section 1) of Example 8 and according to the ingredient compositions and contents shown in Table 6 below. The uncoated tablets were placed in a Hi-coater (SFC-30N, Sejong Machinery Co., Ltd., South Korea). According to the ingredient contents shown in Table 6, hypromellose phthalate (HP-55) and polyethylene glycol 6000 were dissolved and dispersed to prepare a coating solution which was then coated thereon to prepare losartan-containing delayed-release tablets.

### 2) Preparation of hepatitis-preventing and inhibiting agent prior-release granules

According to the ingredient compositions and contents shown in Table 6 below, L-glutamine, a ginko biloba extract, arginine sodium, microcrystalline cellulose, pregelatinized starch, and corn starch were sieved through a No. 35 sieve and mixed in a high-speed mixer. Meanwhile, hydroxypropylcellulose was dissolved in purified water (80 mg/tablet) to prepare a binding solution. The binding solution and the mixture of main ingredients were placed in a high-speed mixer, followed by kneading. After completion of the kneading process, the kneaded material was granulated using an oscillator with a No. 18 sieve, and the granules were dried in a hot-water dryer at 30°C. After completion of the drying process, the granules were sieved again through an F-type grinder equipped with a No. 20 sieve to prepare hepatitis-preventing and inhibiting agent prior-release granules.

### 3) Mixing and capsule filling

The final products of Sections 1) and 2) were filled in No. 1 capsules using a capsule filling machine, thereby preparing a formulation in the form of a capsule.

### <Example 45> Preparation of capsules (tablets-tablets)

### 1) Preparation of irbesartan delayed-release film-coated tablets

Irbesartan-containing film-coated tablets were prepared in the same manner as in Example 2 and according to the ingredient compositions and contents shown in Example 45 of Table 6 below.

### 2) Preparation of hepatitis-preventing and inhibiting agent prior-release tablets

Hepatitis-preventing and inhibiting agent prior-release pellets were prepared in the same manner as in Section 2) of Example 41 and according to the ingredient compositions and contents shown in Example 45 of Table 6 below, except that erdosteine, cholecalciferol and butylated hydroxyanisole were used in place of hymecromone.

### 3) Mixing and capsule filling

The final products of Sections 1) and 2) were filled in capsules using a capsule filling machine, thereby preparing a formulation in the form of a capsule.

**Table 1**

| Ingredients (product name, manufacturer) | | Composition ratio (mg/unit formulation) | | | | | | |
|---|---|---|---|---|---|---|---|---|
| | | Example No. | | | | | | |
| | | 1 | 2 | 3 | 4 | 5 | 6 | 7 |
| Angiotensin-II-receptor blocker-containing ingredient | Losartan potassium (Cadila) | | | | 50 | 50 | | |
| | Irbesartan (Ranbaxy) | | 150 | | | | | |
| | Valsartan (Dr. Reddy's) | | | 80 | | | | |
| | Telmisartan (Ranbaxy) | 40 | | | | | | 40 |
| | Eprosartan (MSN) | | | | | | 600 | |
| | Microcrystalline cellulose (Vivapur 12, JRS) | 123 | 45 | 60 | 100 | 100 | 100 | 123 |
| | Pregelatinized starch (Starch 1500, Colorcon) | | 45 | | | | | |
| | Sodium starch glycolate (Explotab, JRS) | | | | 18 | 18 | 18 | |
| | Polyvinylpyrrolidone copolymer (PVP VA64Fine, BASF) | | | | | | | |
| | Hydroxypropylcellulose (HPC-L, Hercules) | | | | 4 | 4 | 4 | |
| | Lactose (Lactose 200, DMV) | | 30 | | 30 | 30 | 30 | |
| | Croscarmellose sodium (Vivasol, JRS PHARMA) | | 15 | | | | | |
| | Crosslinked polyvinylpyrrolidone (Kollidon CL, BASF) | | | 15 | | | | |
| | Poloxamer 188 (Lutrol F68, BASF) | | 9 | | | | | |
| | Polyvinylpyrrolidone (PVP, BASF) | 7 | | 10 | | | | 7 |
| | Sorbitol (Sorbitol powder, Roquette) | 10 | | | | | | 10 |
| | NaOH (Duksan) | 3 | | | | | | 3 |
| | Meglumine (USP, Spectrum Chemical&Laboratory Products) | 1 | | | | | | 1 |
| | Colloidal silicon dioxide (Aerosil pharma 200, Degussa) | | 3 | | 2 | 2 | 2 | |
| | Polyvinyl acetate (Kollicoat SR30D, BASF) | 24 | | | | | | 24 |
| | Hypromellose (Pharmacoat 615, Shin-Etsu) | | | 7 | | | | |
| | Hypromellose phthalate HP-55 (HPMC-P 55, Shin-Etsu) | | | 12 | 67.3 | 67.7 | 67.6 | |
| | Polyethylene glycol 6000 (PEG 6000, Duksan) | | | | 6.7 | 6.7 | 6.8 | |
| | Cellulose acetate (acetyl group 32.0%)(Eastman cellulose acetate, Eastman) | | 40 | | | | | |
| | Cellulose acetate (acetyl group 39.8%)(Eastman cellulose acetate, Eastman) | | 40 | | | | | |
| Post-mixing | Magnesium stearate (Nof) | 1 | 1 | 1 | 1 | 1 | 1 | 1 |
| Film-coated layer | Hypromellose 2910 (Pharmacoat 603, Shin-Etsu) | | | | | 12.4 | 31.6 | 9.9 |
| | Hydroxypropylcellulose (HPC-L, Hercules) | | | | | 12.4 | 31.6 | 9.9 |
| | Titanium oxide (Fanglian, JHP) | | | | | 1.8 | 4.6 | 1.4 |
| | Talc (Nippon Soda) | | | | | 1 | 2.8 | 0.8 |
| Total | | 209 | 378 | 185 | 279 | 307 | 900 | 231 |

**Table 2**

| Ingredients (product name, manufacturer) | | Composition ratio (mg/unit formulation) | | | | | | |
|---|---|---|---|---|---|---|---|---|
| | | Example No. | | | | | | |
| | | 8 | 9 | 10 | 11 | 12 | 13 | 14 |
| Angiotensin-II-receptor blocker-containing ingredient | Losartan potassium (Cadila) | 50 | 50 | 50 | 50 | 50 | 50 | 50 |
| | Microcrystalline cellulose (Vivapur 12, JRS) | 14 | 14 | 14 | 14 | 14 | 14 | 14 |
| | Pregelatinized starch (Starch 1500, Colorcon) | 10 | 10 | 10 | 10 | 10 | 10 | 10 |
| | Polyvinylpyrrolidone copolymer (PVP VA64Fine, BASF) | 4.5 | 4.5 | 4.5 | 4.5 | 4.5 | 4.5 | 4.5 |
| | Colloidal silicon dioxide (Aerosil pharma 200, Degussa) | 1 | 1 | 1 | 1 | 1 | 1 | 1 |
| | Magnesium stearate (Nof) | 1.5 | 1.5 | 1.5 | 1.5 | 1.5 | 1.5 | 1.5 |
| Delayed-release coating layer | Polyvinylpyrrolidone (PVP, BASF) | | | | | 4 | 8 | 12 |
| | Ethylcellulose (Ethocel, Colorcon) | 8 | 12 | 16 | 20 | 16 | 16 | 16 |
| | Acryl-EZE (Colorcon) | 8 | 8 | 8 | 8 | 8 | 8 | 8 |
| Film-coated layer | Hypromellose 2910 (Pharmacoat 603, Shin-Etsu) | 3.6 | 3.6 | 4 | 4 | 4 | 4 | 4 |
| | Hydroxypropylcellulose (HPC-L, Hercules) | 3.6 | 3.6 | 4 | 4 | 4 | 4 | 4 |
| | Titanium oxide (Fanglian, JHP) | 0.5 | 0.5 | 0.7 | 0.7 | 0.7 | 0.7 | 0.7 |
| | Talc (Nippon Soda) | 0.3 | 0.3 | 0.3 | 0.3 | 0.3 | 0.3 | 0.3 |
| Total | | 105 | 109 | 114 | 118 | 118 | 122 | 126 |

**Table 3**

| Ingredients (product name, manufacturer) | | Composition ratio (mg/unit formulation) | | | | | | | | | |
|---|---|---|---|---|---|---|---|---|---|---|---|
| | | Example No. | | | | | | | | | |
| | | 15 | 16 | 17 | 18 | 19 | 20 | 21 | 22 | 23 | 24 |
| Angiotensin-II-receptor blocker-containing ingredient | Losartan potassium (Cadila) | | 50 | 50 | | | | 50 | 50 | | |
| | Olmesartan medoxomil (MSN) | 20 | | | | | | | | | |
| | Irbesartan (Ranbaxy) | | | | | 150 | | | | | |
| | Valsartan (Dr. Reddy's) | | | | | | 80 | | | | |
| | Telmisartan (Ranbaxy) | | | | 40 | | | | | | 40 |
| | Eprosartan (MSN) | | | | | | | | | 600 | |
| | Alpha lipoic acid (USP, Spectrum Chemical&Laboratory Products) | | | | 200 | | | | | | |
| | β-carotene (SPECTRUM LABORATORY PRODUCTS INC.) | | | | | 10 | | | | | |
| | Silymarin (Alchem International) | | | | | | 70 | | | | |
| | Biphenyldimethyldicarboxylate (Daewoong Bio) | | | | | | | 25 | | | |
| | Selenized yeast (Won Poong Pharmaceutical) | | | | | | | | | 92 | |
| | Urazamide (Yuhan Chemical) | | | | | | | | | | |
| | Fraction flavonoid purifiee micronise (Kukjeon Pharmaceutical) | | | | | | | | | | |
| | Cicloxilic acid (Jisuchem) | | | | | | | | | | |
| | Riboflavin tetrabutyrate (Hwaduk Pharmaceutical) | | | | | | | | | | |
| | Ascorbic acid (Roche) | | | | | | | | | | |
| | Pyridoxamine hydrochloride (Chem-Impex) | | | | | | | | | | |
| | Agaro-oligosaccharide (Takara Bio) | | | | | | | | | | |
| | Melatonin (Acros Organics) | | | | | | | | | | |
| | Ursodeoxycholic acid (Daewoong Bio) | | | | | | | | | | |
| | Naphthyl acetic acid (Advanced Synthesis) | | | | | | | | | | |
| | Azintamide (DONGBANG FTL) | | | | | | | | | | |
| | Microcrystalline cellulose (Vivapur 12,JRS) | 123 | | 14 | 123 | 45 | 60 | 100 | 100 | 100 | 123 |
| | Pregelatinized starch (Starch 1500, Colorcon) | 20 | | 10 | | 45 | | | | | |
| | Sodium starch glycolate (Explotab, JRS) | | | | | | | 18 | 18 | 18 | |
| | Polyvinylpyrrolidone copolymer (PVP VA64Fine, BASF) | | | 4.5 | | | | | | | |
| | Hydroxypropylcellulose (HPC-L, Hercules) | 3 | | | | | | 4 | 4 | 4 | |
| | Lactose (Lactose 200, DMV) | | | | | 30 | | 30 | 30 | 30 | |
| | Croscarmellose sodium (Vivasol, JRS PHARMA) | | | | | 15 | | | | | |
| | Crosslinked polyvinylpyrrolidone (Kollidon CL, BASF) | | | | | | 15 | | | | |
| | Poloxamer 188 (Lutrol F68, BASF) | | | | | 9 | | | | | |
| | Polyvinylpyrrolidone (PVP, BASF) | | | | 7 | | 7 | | | | 7 |
| | Sorbitol (Sorbitol powder, Roquette) | | | | 10 | | | | | | 10 |
| | NaOH (Duksan) | | | | 3 | | | | | | 3 |
| | Meglumine (USP, Spectrum Chemical&Laboratory Products) | | | | 1 | | | | | | 1 |
| | Sugar seed (Non-pareil 101, Freund) | | 35 | | | | | | | | |
| | Polyvinyl acetate (Kollicoat SR30D, BASF) | 24 | | | 24 | | | | | | 24 |
| | Hypromellose (Pharmacoat 615, Shin-Etsu) | | 5 | | | | 7 | | | | |
| | Hypromellose phthalate HP-55 (HPMC-P 55, Shin-Etsu) | | 45 | 7.2 | | | 12 | 67.3 | 67.6 | 67.6 | |
| | Polyethylene glycol 6000 (PEG 6000, Duksan) | | | 0.8 | | | | 6.7 | 6.8 | 6.8 | |
| | Cellulose acetate (acetyl group 32.0%)(Eastman cellulose acetate, Eastman) | | | | | 40 | | | | | |
| | Cellulose acetate (acetyl group 39.8%)(Eastman cellulose acetate, Eastman) | | | | | 40 | | | | | |
| | Colloidal silicon dioxide (Aerosil pharma 200, Degussa) | | | 1 | | | | 2 | 2 | | |
| | Magnesium stearate (Nof) | | | 1.5 | | | | | | | |
| Post-mixing | Colloidal silicon dioxide (Aerosil pharma 200, Degussa) | | | | | 3 | | | | 2 | |
| | Magnesium stearate (Nof) | 1 | 1 | 1 | 1 | | 1 | 1 | 1 | 1 | 1 |
| Film-coated layer | Acetylcysteine (Apollo Scientific) | | | | | | | | 100 | | |
| | ASTO (Coriolus versicolor polysaccharide, Hwail Pharmaceutical) | | | | | | | | | 240 | |
| | Citiolone (Facus Pharmaceutical) | | | | | | | | | | 200 |
| | Hypromellose 2910 (Pharmacoat 603, Shin-Etsu) | | | | | | | | 13.3 | 14.8 | 24.7 |
| | Hydroxypropylcellulose (HPC-L, Hercules) | | | | | | | | 13.3 | 14.8 | 24.7 |
| | Titanium oxide (Fanglian, JHP) | | | | | | | | 1.8 | 2.3 | 3.8 |
| | Talc (Nippon Soda) | | | | | | | | 1.2 | 1.7 | 2.8 |
| Total | | 191 | 136 | 89 | 409 | 388 | 252 | 304 | 409 | 1195 | 465 |

**Table 4**

| Ingredients (product name, manufacturer) | | Composition ratio (mg/unit formulation) | | | | | | |
|---|---|---|---|---|---|---|---|---|
| | | Example No. | | | | | | |
| | | 25 | 26 | 27 | 28 | 29 | 30 | 31 |
| Angiotensin-II-receptor blocker-containing ingredient | Losartan potassium (Cadila) | 50 | 50 | 50 | 50 | 50 | 50 | 50 |
| | Urazamide (Yuhan Chemical) | 200 | | | | | | |
| | Fraction flavonoid purifiee micronise (Kukjeon Pharmaceutical) | | 100 | | | | | |
| | Cicloxilic acid (Jisuchem) | | | 40 | | | | |
| | Riboflavin tetrabutyrate (Hwaduk Pharmaceutical) | | | | 20 | | | |
| | Ascorbic acid (Roche) | | | | 15 | | | |
| | Pyridoxamine hydrochloride (Chem-Impex) | | | | 25 | | | |
| | Agaro-oligosaccharide (Takara Bio) | | | | | 100 | | |
| | Melatonin (Acros Organics) | | | | | 0.1 | | |
| | Ursodeoxycholic acid (Daewoong Bio) | | | | | | 5 | |
| | Naphthyl acetic acid (Advanced Synthesis) | | | | | | | 15 |
| | Azintamide (DONGBANG FTL) | | | | | | | 50 |
| | Microcrystalline cellulose (Vivapur 12, JRS) | 14 | 14 | 14 | 14 | 14 | 14 | 14 |
| | Pregelatinized starch (Starch 1500, Colorcon) | 10 | 10 | 10 | 10 | 10 | 10 | 10 |
| | Polyvinylpyrrolidone copolymer (PVP VA64Fine, BASF) | 4.5 | 4.5 | 4.5 | 4.5 | 4.5 | 4.5 | 4.5 |
| | Polyvinylpyrrolidone (PVP, BASF) | | | | | 4 | 8 | 10 |
| | Colloidal silicon dioxide (Aerosil pharma 200, Degussa) | 1 | 1 | 1 | 1 | 1 | 1 | 1 |
| | Magnesium stearate (Nof) | 1.5 | 1.5 | 1.5 | 1.5 | 1.5 | 1.5 | 1.5 |
| Film-coated layer | Ethylcellulose (Ethocel, Colorcon) | 8 | 12 | 16 | 20 | 16 | 16 | 16 |
| | Acryl-EZE (Colorcon) | 8 | 8 | 8 | 8 | 8 | 8 | 8 |
| Total | | 297 | 201 | 145 | 169 | 209.1 | 118 | 180 |

**Table 5**

| Ingredients (product name, manufacturer) | | Composition ratio (mg/unit formulation) | | | | | | |
|---|---|---|---|---|---|---|---|---|
| | | Example No. | | | | | | |
| | | 32 | 33 | 34 | 35 | 36 | 37 | 38 |
| Delayed-release compartment | Losartan potassium (Cadila) | | 50 | 100 | | | 50 | |
| | Valsartan (Dr. Reddy's) | 80 | | | | | | |
| | Telmisartan (Ranbaxy) | | | | | | | 40 |
| | Eprosartan (MSN) | | | | | 600 | | |
| | Candesartan cilexetil (Ranbaxy) | | | | 16 | | | |
| | Irbesartan (Ranbaxy) | | | | | | | |
| | Microcrystalline cellulose (Vivapur 12, JRS) | 40 | 50 | 150 | | 100 | 14 | 123 |
| | Pregelatinized starch (Starch 1500, Colorcon) | | | | | | 10 | |
| | Polyvinylpyrrolidone copolymer (PVP VA64Fine, BASF) | | | | | | 4.5 | |
| | Sodium starch glycolate (Explotab, JRS) | | 5 | 8 | | 18 | | |
| | Hydroxypropylcellulose (HPC-L, Hercules) | | 5 | 8 | 2 | 4 | | |
| | Lactose (Lactose 200, DMV) | | 25 | 30 | 58 | 30 | | |
| | Corn starch (Duksan) | | | | 10 | | | |
| | Carboxymethylcellulose calcium (CMC Ca, Hwawon) | | | | 38 | | | |
| | Crosslinked polyvinylpyrrolidone (Kollidon CL, BASF) | 80 | | | | | | |
| | Poloxamer 188 (Lutrol F68, BASF) | | | | | | | |
| | Polyvinylpyrrolidone (PVP, BASF) | 8 | | | | | | 7 |
| | Sorbitol (Sorbitol powder, Roquette) | | | | | | | 10 |
| | NaOH (Duksan) | | | | | | | 3 |
| | Meglumine (USP, Spectrum Chemical&Laboratory Products) | | | | | | | 1 |
| | Polyvinyl acetate (Kollicoat SR30D, BASF) | | | | | 7 | | |
| | Hypromellose (Pharmacoat 615, Shin-Etsu) | 3.2 | 2 | | | | 1 | |
| | Hypromellose phthalate HP-50 (HPMC-P 50, Shin-Etsu) | | | 60 | | 12 | | |
| | Hypromellose phthalate HP-55 (HPMC-P 55, Shin-Etsu) | | | | | | | 7.2 |
| | Polyethylene glycol 6000 (PEG 6000, Duksan) | | | 6 | 15 | | | 0.8 |
| | Cellulose acetate (acetyl group 32.0%)(Eastman cellulose acetate, Eastman) | 32 | 20 | | | | | |
| | Cellulose acetate (acetyl group 39.8%)(Eastman cellulose acetate, Eastman) | 32 | 20 | | | | | |
| | Hypromellose acetate succinate (HPMC-AS LF, Shin-Etsu) | | | | 55 | | | |
| | Acryl-EZE (Colorcon) | | | | | | 8 | |
| | Colloidal silicon dioxide (Aerosil pharma 200, Degussa) | | | | | | 1 | |
| | Magnesium stearate (Nof) | | | | 1 | 1 | 1.5 | 1 |
| Post-mixing | Magnesium stearate (Nof) | 4.8 | 3 | 1 | | | | |
| Prior-release compartment | Haematoporphyrin (Atomole Scientific) | 3 | | | | | | |
| | Ubidecarenone (Daewoong Bio) | 10 | | | | | | |
| | Arginine hydrochloride (ILSHIN CHEMICAL) | | 125 | | | | | |
| | Ornithine hydrochloride (CKD BIO) | | 13 | | | | | |
| | Erdosteine (Ilsung Pharmaceuticals) | | | 150 | | | | |
| | Guaiacol (AOKchem) | | | 4 | | | | |
| | Creatine (Shanghai Orgchem) | | | 50 | | | | |
| | Riboflavin | | | | 5 | | | |
| | Cholecalciferol | | | | 5 | | | |
| | L-glutathione | | | | | 10 | | |
| | L-glutamine | | | | | 5 | | |
| | Sodium selenite | | | | | | 20 | |
| | Ginko biloba extract | | | | | | 10 | |
| | Butylated hydroxyanisole | | | | | | | 3 |
| | L-ornithine | | | | | | | 12 |
| | Microcrystalline cellulose (Vivapur 12, JRS) | 100 | 100 | 100 | 100 | 100 | 100 | 100 |
| | Pregelatinized starch (Starch 1500, Colorcon) | 50 | 50 | 50 | 50 | 50 | 50 | 50 |
| | Corn starch (Duksan) | 50 | 50 | 50 | 50 | 50 | 50 | 50 |
| | Hydroxypropylcellulose (HPC-L, Hercules) | 6 | 6 | 6 | 6 | 6 | 6 | 6 |
| | Magnesium stearate (Nof) | | | | 1 | 1 | 1 | 1 |
| Post-mixing | Magnesium stearate (Nof) | 1 | 1 | 1 | | | | |
| Coating layer | Hypromellose 2910 (Pharmacoat 603, Shin-Etsu) | | | 30 | | 39.5 | | 17.5 |
| | Hydroxypropylcellulose (HPC-L, Hercules) | | | 30 | | 39.5 | | 17.5 |
| | Titanium oxide (Fanglian, JHP) | | | 4.4 | | 5.7 | | 2.5 |
| | Talc (Nippon Soda) | | | 2.6 | | 3.3 | | 1.5 |
| Total | | 500 | 525 | 841 | 412 | 1082 | 327 | 454 |

**Table 6**

| Ingredients (product name, manufacturer) | | Composition ratio (mg/unit formulation) | | | | | | |
|---|---|---|---|---|---|---|---|---|
| | | Example No. | | | | | | |
| | | 39 | 40 | 41 | 42 | 43 | 44 | 45 |
| Delayed-release compartment | Losartan potassium (Cadila) | | | | 50 | | 50 | |
| | Valsartan (Dr. Reddy's) | | | 80 | | | | |
| | Telmisartan (Ranbaxy) | | | | | | | |
| | Olmesartan medoxomil (MSN) | | 20 | | | | | |
| | Eprosartan (MSN) | | | | | 600 | | |
| | Candesartan cilexetil (Ranbaxy) | 16 | | | | | | |
| | Irbesartan (Ranbaxy) | | | | | | | 150 |
| | Microcrystalline cellulose (Vivapur 12, JRS) | 20 | 123 | 60 | | | 14 | 45 |
| | Pregelatinized starch (Starch 1500, Colorcon) | | 10 | | | | 10 | 45 |
| | Polyvinylpyrrolidone copolymer (PVP VA64Fine, BASF) | | | | | | 4.5 | |
| | Hydroxypropylcellulose (HPC-L, Hercules) | | 4 | | | | | |
| | Lactose (Lactose 200, DMV) | | | | | | | 30 |
| | Croscarmellose sodium (Vivasol, JRS PHARMA) | | | | | | | 15 |
| | | | | | | | | |
| | Crosslinked polyvinylpyrrolidone (Kollidon CL, BASF) | | | 15 | | | | |
| | Poloxamer 188 (Lutrol F68, BASF) | | | | | | | 9 |
| | Polyvinylpyrrolidone (PVP, BASF) | | | 7 | | | | |
| | Sugar seed (Non-pareil 101, Freund) | | | | 35 | 35 | | |
| | Polyvinyl acetate (Kollicoat SR30D, BASF) | | 24 | | | | | |
| | Hypromellose (Pharmacoat 615, Shin-Etsu) | | | 7 | 5 | 5 | | |
| | Hypromellose phthalate HP-55 (HPMC-P 55, Shin-Etsu) | | | 12 | 45 | 45 | 7.2 | |
| | Polyethylene glycol 6000 (PEG 6000, Duksan) | | | | | | 0.8 | |
| | Cellulose acetate (acetyl group 32.0%)(Eastman cellulose acetate, Eastman) | | | | | | | 40 |
| | Cellulose acetate (acetyl group 39.8%)(Eastman cellulose acetate, Eastman) | | | | | | | 40 |
| | Ethylcellulose (Ethocel, Colorcon) | 5 | | | | | | |
| | Sodium chloride | 3 | | | | | | |
| | Colloidal silicon dioxide (Aerosil pharma 200, Degussa) | | | | | | 1 | 3 |
| | Magnesium stearate (Nof) | 1 | | | | | 1.5 | 1 |
| Prior-release compartment | Haematoporphyrin (Atomole Scientific) | | | | 6 | | | |
| | Erdosteine (Ilsung Pharmaceuticals) | | | | | | | 5 |
| | Guaiacol (AOKchem) | | | | | 10 | | |
| | Cholecalciferol | | | | | 10 | | 3 |
| | L-glutathione | | | | | | 15 | |
| | Ginko biloba extract | | | | 5 | | 3 | |
| | Butylated hydroxyanisole | | | | | | | 3 |
| | Arginine sodium | 10 | | | | 10 | 3 | |
| | Protoporphyrin disodium | | 10 | | | | | |
| | Hymecromone | | | 20 | | | | |
| | Sugar seed (Non-pareil 101, Freund) | | | | 10 | | | |
| | Microcrystalline cellulose (Vivapur 12, JRS) | 100 | 100 | 100 | | 100 | 100 | 100 |
| | Pregelatinized starch (Starch 1500, Colorcon) | 50 | 50 | 50 | | 50 | 50 | 50 |
| | Corn starch | 50 | 50 | 50 | | 50 | 50 | 50 |
| | Hydroxypropylcellulose (HPC-L, Hercules) | 6 | 6 | 6 | 6 | 6 | 6 | 6 |
| | Magnesium stearate (Nof) | 1 | | 1 | | 1 | | 1 |
| Post-mixing | Magnesium stearate (Nof) | | 2 | | | | | |
| Coating layer | Hypromellose 2910 (Pharmacoat 603, Shin-Etsu) | 11.7 | | | | | | |
| | Hydroxypropylcellulose (HPC-L, Hercules) | 11.7 | | | | | | |
| | Titanium oxide (Fanglian, JHP) | 1.6 | | | | | | |
| | Talc (Nippon Soda) | 1 | | | | | | |
| Total | | 288 | 399 | 408 | 162 | 922 | 316 | 596 |

### Experimental Example 1: Comparative dissolution profile test

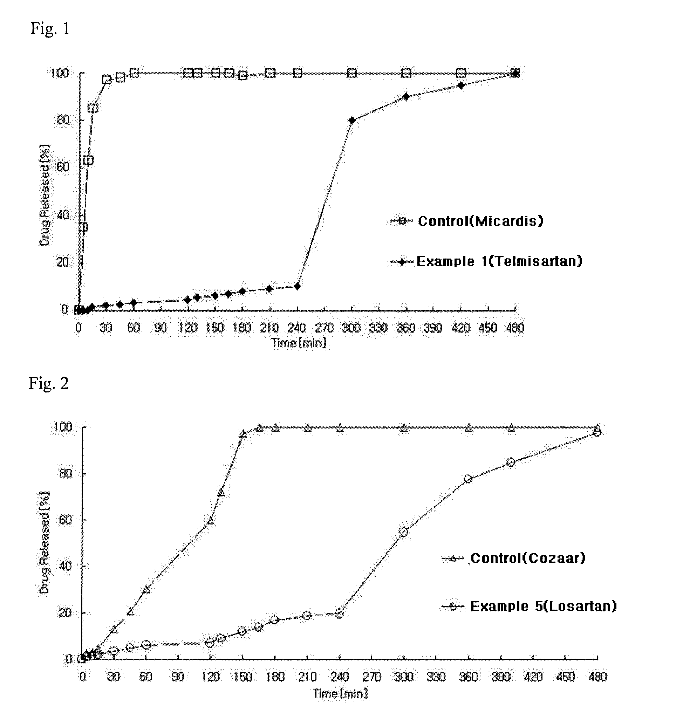

A comparative dissolution profile test was performed using a telmisartan delayed-release uncoated tablet prepared in Example 1 and a control drug (Micardis, Boehringer Ingelheim: telmisartan single drug). The dissolution profile test of the telmisartan ingredient was performed for a total of 480 minutes, in which the dissolution medium was changed from a simulated gastric juice to a simulated intestinal juice 120 minutes after the start of the test. Details of the dissolution profile test of each ingredient are as follows. The results obtained are shown in FIG. 1. In FIG. 1, the x-axis represents the time series (min), and the y-axis represents the dissolution rate (Drug Released, %).

As can be seen in FIG. 1, when the dissolution profile test was performed, the telmisartan ingredient of the uncoated tablet of Example 1 showed a very slow dissolution rate as compared to that of the control drug Micardis, and showed the drug dissolution of less than 20% up to a total of 4 hours.

As described above, the initial release of telmisartan in the telmisartan delayed-release uncoated tablet is very slow, unlike dissolution profiles of the telmisartan single drug as a control drug. Therefore, telmisartan can be absorbed during the night upon evening administration of the tablet, so it can be seen that the inventive tablet is highly effective in the treatment of hypertension.
Test Method for angiotensin-II-receptor blocker: Based on the general dissolution test method described in the Korean Pharmacopoeia (8^{th} revision)
Test method: Paddle method, 50 rpm
Dissolution medium: 0.01 M hydrochloric acid solution 750 mL (simulated gastric juice), pH 6.8 phosphate buffer 1,000 mL (simulated intestinal juice)
Analysis method: UV-Vis spectrophotometry

### Experimental Example 2: Comparative dissolution profile test

A comparative dissolution profile test was performed using a losartan-containing film-coated tablet prepared in Example 5 and a control drug (Cozaar, MSD: losartan single drug). Details of the dissolution profile test of each ingredient are the same as those of Experimental Example 1. The results obtained are shown in FIG. 2. In FIG. 2, the x-axis represents the time series (min), and the y-axis represents the dissolution rate (Drug Released, %).

As can be seen in FIG 2, when the dissolution profile test was performed under the conditions of Experimental Example 1, the losartan ingredient of the delayed-release granule-containing film-coated tablet showed a very slow dissolution rate as compared to that of the control drug Cozaar, and showed the drug dissolution of less than 20% up to a total of 240 minutes.

As described above, the initial release of losartan in the losartan-containing film-coated tablet of the present invention is significantly slow, unlike dissolution profiles of the losartan single drug as a control drug. Therefore, losartan can be absorbed during the night upon evening administration of the tablet, so it can be seen that the inventive tablet is highly effective in the treatment of hypertension.

### Experimental Example 3: Comparative dissolution profile test

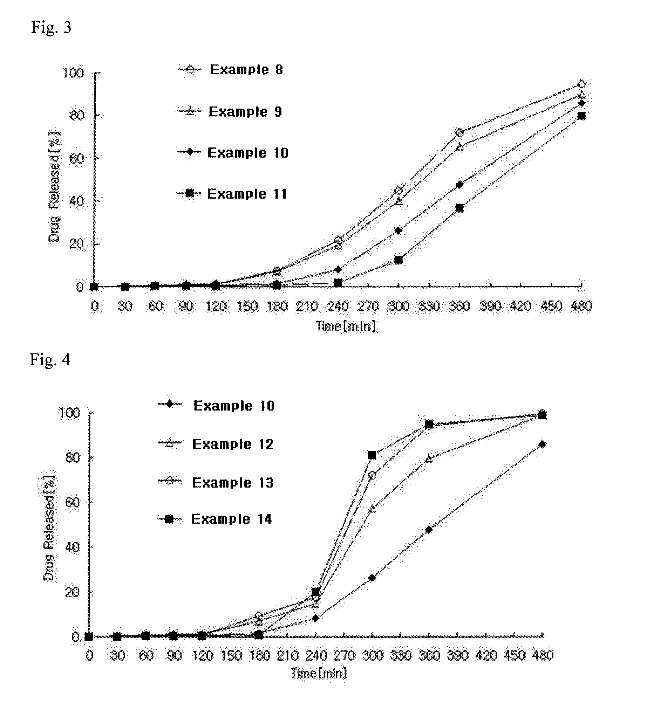

A comparative dissolution profile test was performed using film-coated tablets of Examples 8 to 11. Details of the dissolution profile test are the same as those of Experimental Example 1. The results obtained are shown in FIG. 3. In FIG. 3, the x-axis represents the time series (min), and the y-axis represents the dissolution rate (Drug Released, %).

As can be seen in FIG. 3, when the dissolution profile test was performed under the conditions of Experimental Example 1, the dissolution rate of losartan was less than 20% up to a total of 240 minutes in the delayed-release film-containing film-coated tablet of the present invention, thus exhibiting a significant decrease in dissolution rate of the losartan ingredient in response to an increase in the amount of ethylcellulose to be used. That is, coating of the tablet with ethylcellulose resulted in significantly delayed dissolution of the losartan ingredient.

Therefore, the initial release of losartan in the losartan film-coated tablet containing a delayed-release film can be delayed up to the intended time by controlling the amount of ethylcellulose coated. Therefore, losartan can be absorbed during the night upon evening administration of the tablet, so it can be seen that the inventive tablet is highly effective in the treatment of hypertension.

### Experimental Example 4: Comparative dissolution profile test

A comparative dissolution profile test was performed using formulations of Examples 10, and 12 to 14. Details of the dissolution profile test are the same as those of Experimental Example 1. The results obtained are shown in FIG. 4. In FIG. 4, the x-axis represents the time series (min), and the y-axis represents the dissolution rate (Drug Released, %).

As can be seen in FIG. 4, when the dissolution profile test was performed under the conditions of Experimental Example 1, the delayed-release film-containing film-coated tablet of the present invention showed relatively rapid release of the losartan ingredient after an intended lag time when crosslinked polyvinylpyrrolidone is incorporated in the ethylcellulose-coated delayed-release layer. The dissolution rate of the losartan ingredient was less than 20% up to a total of 240 minutes and the losartan ingredient was rapidly released with an increase in the amount of crosslinked polyvinylpyrrolidone to be used.

Therefore, the losartan film-coated tablet containing a delayed-release film can achieve rapid release of losartan after an intended lag time by controlling the content of crosslinked polyvinylpyrrolidone in the ethylcellulose-coated delayed-release layer. Therefore, losartan can be absorbed during the night upon evening administration of the tablet, so it can be seen that the inventive tablet is highly effective in the treatment of hypertension.

### Experimental Example 5: Comparative dissolution profile test

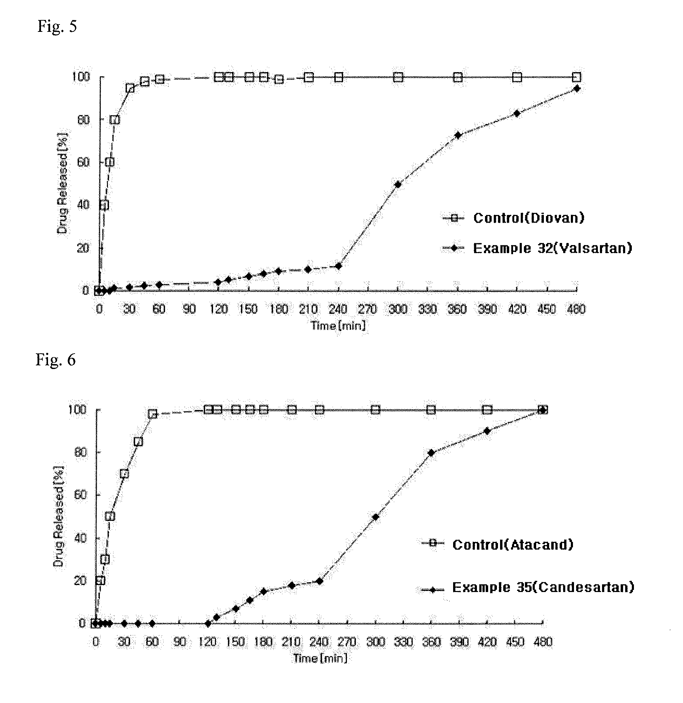

A comparative dissolution profile test was performed using a multi-layered tablet containing a valsartan delayed-release layer prepared in Example 32 and a control drug (Diovan, MSD: valsartan single drug). Details of the dissolution profile test are the same as those of Experimental Example 1. The results obtained are shown in FIG. 5. In FIG. 5, the x-axis represents the time series (min), and the y-axis represents the dissolution rate (Drug Released, %).

As can be seen in FIG. 5, when the dissolution profile test was performed under the conditions of Experimental Example 1, the valsartan ingredient of the delayed-release layer-containing multi-layered tablet of the present invention showed a very slow dissolution rate as compared to that of the control drug Diovan. In the dissolution profile test results for the valsartan ingredient, the dissolution rate of the valsartan ingredient was about 20% up to a total of 240 minutes in the valsartan delayed-release layer-containing multi-layered tablet of the present invention, which was far lower than that of the control drug.

As described above, the initial release of valsartan in the valsartan delayed-release layer-containing multi-layered tablet is significantly slow, unlike dissolution profiles of the valsartan single drug as a control drug. Therefore, valsartan can be absorbed during the night upon evening administration of the tablet, so it can be seen that the inventive tablet is highly effective in the treatment of hypertension.

### Experimental Example 6: Comparative dissolution profile test

A comparative dissolution profile test was performed using a candesartan cilexetil press-coated tablet prepared in Example 35 and a control drug (Atacand, AstraZeneca: candesartan single drug). Details of the dissolution profile test are the same as those of Experimental Example 1. The results obtained are shown in FIG. 6. In FIG. 6, the x-axis represents the time series (min), and the y-axis represents the dissolution rate (Drug Released, %).

As can be seen in FIG. 6, when the dissolution profile test was performed under the conditions of Experimental Example 1, the candesartan ingredient of the press-coated tablet of the present invention showed a very slow dissolution rate of about 20% up to a total of 240 minutes, as compared to that of the control drug Atacand. As described above, the initial release of losartan in the candesartan cilexetil press-coated tablet is significantly slow, unlike dissolution profiles of the candesartan single drug as a control drug. Therefore, candesartan can be absorbed during the night upon evening administration of the tablet, so it can be seen that the inventive tablet is highly effective in the treatment of hypertension.

### Experimental Example 7: Comparative dissolution profile test

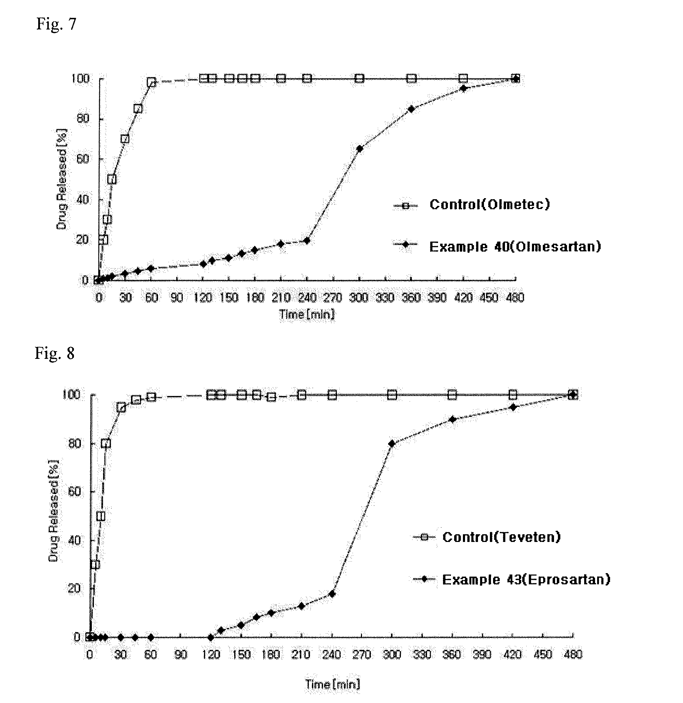

A comparative dissolution profile test was performed using an olmesartan medoxomil capsule (granule) prepared in Example 40 and a control drug (Olmetec, Daewoong Pharmaceutical: olmesartan medoxomil single drug). Details of the dissolution profile test are the same as those of Experimental Example 1. The results obtained are shown in FIG. 7. In FIG. 7, the x-axis represents the time series (min), and the y-axis represents the dissolution rate (Drug Released, %).

As can be seen in FIG. 7, when the dissolution profile test was performed under the conditions of Experimental Example 1, the olmesartan ingredient of the capsule (granule) of the present invention showed a very slow dissolution rate of about 20% up to a total of 240 minutes, as compared to that of the control drug Olmetec. As described above, the initial release of olmesartan in the olmesartan medoxomil capsule (granule) is significantly slow, unlike dissolution profiles of the losartan single drug as a control drug. Therefore, olmesartan can be absorbed during the night upon evening administration of the capsule, so it can be seen that the inventive capsule is highly effective in the treatment of hypertension.

### Experimental Example 8: Comparative dissolution profile test

A comparative dissolution profile test was performed using an eprosartan capsule (pellet) prepared in Example 43 and a control drug (Teveten, Abbott: eprosartan single drug). Details of the dissolution profile test are the same as those of Experimental Example l. The results obtained are shown in FIG. 8. In FIG. 8, the x-axis represents the time series (min), and the y-axis represents the dissolution rate (Drug Released, %).

As can be seen in FIG. 8, when the dissolution profile test was performed under the conditions of Experimental Example 1, the eprosartan ingredient of the capsule (pellet) of the present invention showed a very slow dissolution rate, as compared to that of the control drug Teveten. As described above, the initial release of eprosartan in the eprosartan capsule (pellet) is significantly slow, unlike dissolution profiles of the eprosartan single drug as a control drug. Therefore, eprosartan can be absorbed during the night upon evening administration of the capsule, so it can be seen that the inventive capsule is highly effective in the treatment of hypertension.

### Experimental Example 9: Comparative dissolution profile test

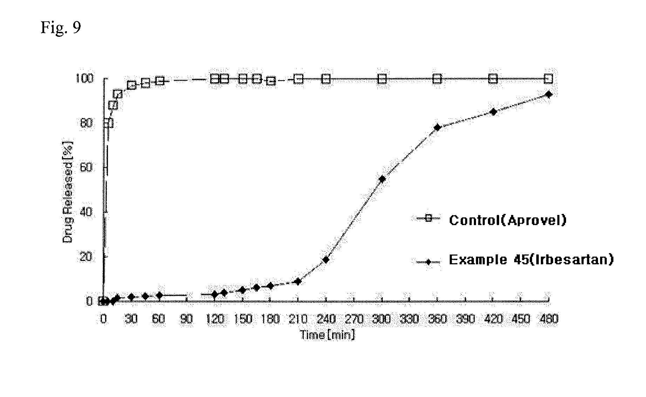

A comparative dissolution profile test was performed using an irbesartan capsule (tablet) prepared in Example 45 and a control drug (Aprovel, MSD: irbesartan single drug). Details of the dissolution profile test of each ingredient are the same as those of Experimental Example 1. The results obtained are shown in FIG. 9. In FIG. 9, the x-axis represents the time series (min), and the y-axis represents the dissolution rate (Drug Released, %).

As can be seen in FIG. 9, when the dissolution profile test was performed under the conditions of Experimental Example 1, the irbesartan ingredient of the capsule (tablet) of the present invention showed a very slow dissolution rate, as compared to that of the control drug Diovan. In the dissolution profile test results for the irbesartan ingredient, the dissolution rate of the irbesartan ingredient was about 20% up to a total of 240 minutes in the irbesartan capsule (tablet) of Example 45, which was far lower than that of the control drug. As described above, the initial release of irbesartan in the irbesartan capsule (tablet) of the present invention is significantly slow, unlike dissolution profiles of the irbesartan single drug as a control drug. Therefore, irbesartan can be absorbed during the night upon evening administration of the capsule, so it can be seen that the inventive capsule is highly effective in the treatment of hypertension.

### INDUSTRIAL APPLICABILITY

The formulation of the present invention maximizes the effectiveness on pharmacologically and clinically lowering blood pressure and preventing complications when taking the formulation, helps to avoid interactions with a drug which is metabolized by the same enzyme in the liver, and prevents and inhibits the incidence of drug-induced hepatitis which is caused by drug administration for a long time.

## Claims

1. A pharmaceutical formulation comprising an angiotensin-II-receptor blocker as a pharmacologically active ingredient and a release-controlling material.

2. The pharmaceutical formulation according to claim 1, wherein the angiotensin-II-receptor blocker is released at a level of less than 40% by weight within 4 hours after oral administration.

3. The pharmaceutical formulation according to claim 1, wherein the content of the angiotensin-II-receptor blocker in the formulation is in the range of 5 to 600 mg.

4. The pharmaceutical formulation according to any one of claims 1 to 3, wherein the formulation further comprises a hepatitis-preventing and inhibiting agent as a pharmacologically active ingredient.

5. The pharmaceutical formulation according to claim 4, wherein the content of the hepatitis-preventing and inhibiting agent in the formulation is in the range of 0.01 µg to 1 g.

6. The pharmaceutical formulation according to claim 4, wherein the hepatitis-preventing and inhibiting agent is at least one selected from β-carotene, riboflavin, riboflavin tetrabutyrate, riboflavin rocoat, riboflavin phosphate sodium, ascorbic acid, ascorbyl palmitate, calcium ascorbate, nicotinamide ascorbate, sodium ascorbate, dehydroascorbic acid, cholecalciferol, cholecalciferolic acid, ergocalciferol, tocopherol, tocopherol acetate, tocopherol calcium, tocopherol calcium succinate, tocopherol succinate, cysteine, cysteine hydrochloride, cysteine malate, methyl cysteine, carboxymethyl cysteine, methyl cysteine hydrochloride, N-acetyl-L-cysteine, L-glutathione, glutathione disulfide, reduced glutathione, L-glutamine, glutamine hydrochloride, L-glutaminate-L-lysinate, L-glutamine, N(2)-L-alanine-L-glutamine, α-lipoic acid, selenized yeast, selenium, selenium disulfide, sodium selenate, sodium selenite, silymarin, ginko biloba extract, biphenyldimethyldicarboxylate, ursodeoxycholic acid, chenocholic acid, butylated hydroxyanisole, butylated hydroxytoluene, guaiacol, erdosteine, resveratrol, pyridoxamine hydrochloride, pyridoxine hydrochloride, pyridoxal phosphate, agaro-oligosaccharide, fraction flavonoid purifiee micronise, melatonin, ubidecarenone, L-ornithine, ornithine aspartate, ornithine hydrochloride, L-arginine, arginine hydrochloride, arginine sodium, L-arginine monoglutamate, protoporphyrin disodium, haematoporphyrin, haematoporphyrin hydrochloride, Coriolus versicolor polysaccharide, cicloxilic acid, citiolone, urazamide, azintamide, hymecromone, and α-naphthyl acetic acid.

7. The pharmaceutical formulation according to any one of claims 1 to 3, wherein the angiotensin-II-receptor blocker is at least one selected from losartan, valsartan, telmisartan, irbesartan, candesartan, olmesartan, eprosartan, isomers thereof, pharmaceutically acceptable salts thereof, and prodrugs thereof.

8. The pharmaceutical formulation according to any one of claims 1 to 3, wherein the release-controlling material is at least one selected from an enteric polymer, a water-insoluble polymer, a hydrophobic compound, a hydrophilic polymer and a mixture thereof.

9. The pharmaceutical formulation according to any one of claims 1 to 3, wherein the content of the release-controlling material is in the range of 0.1 to 20 parts by weight, relative to 1 part by weight of the pharmacologically active ingredient.

10. The pharmaceutical formulation according to claim 8, wherein the enteric polymer is at least one selected from the group consisting of an enteric cellulose derivative, an enteric acrylic acid copolymer, an enteric polymethacrylate copolymer, an enteric maleic acid copolymer, an enteric polyvinyl derivative, and a mixture thereof.

11. The pharmaceutical formulation according to claim 10, wherein the enteric cellulose derivative is at least one selected from hydroxypropylmethylcellulose acetate succinate, hydroxypropylmethylcellulose phthalate, hydroxymethylethylcellulose phthalate, cellulose acetate phthalate, cellulose acetate succinate, cellulose acetate maleate, cellulose benzoate phthalate, cellulose propionate phthalate, methylcellulose phthalate, carboxymethylethylcellulose, ethylhydroxyethylcellulose phthalate, methylhydroxyethylcellulose and a mixture thereof; the enteric acrylic acid copolymer is at least one selected from a styrene/acrylic acid copolymer, a methyl acrylate/acrylic acid copolymer, a methyl acrylate/methacrylic acid copolymer, a butyl acrylate/styrene/acrylic acid
copolymer, a methyl acrylate/methacrylic acid/octyl acrylate copolymer and a mixture thereof; the enteric polymethacrylate copolymer is at least one selected from a poly(methacrylic acid/methyl methacrylate) copolymer, a poly(methacrylic acid/ethyl acrylate) copolymer and a mixture thereof; the enteric maleic acid copolymer is at least one selected from a vinyl acetate/maleic anhydride copolymer, a styrene/maleic anhydride copolymer, a styrene/maleic monoester copolymer, a vinyl methyl ether/maleic anhydride copolymer, an ethylene/maleic anhydride copolymer, a vinyl butyl ether/maleic anhydride copolymer, an acrylonitrile/methyl acrylate/maleic anhydride copolymer, a butyl acrylate/styrene/maleic anhydride copolymer and a mixture thereof; and the enteric polyvinyl derivative is at least one selected from polyvinylalcohol phthalate, polyvinylacetate phthalate, polyvinylbutyrate phthalate, polyvinylacetacetal phthalate and a mixture thereof.

12. The pharmaceutical formulation according to claim 10, wherein a content of the enteric polymer is in the range of 0.1 parts by weight to 20 parts by weight, relative to 1 part by weight of the pharmacologically active ingredient.

13. The pharmaceutical formulation according to claim 8, wherein the water-insoluble polymer is at least one selected from the group consisting of polyvinyl acetate, a water-insoluble polymethacrylate copolymer, ethylcellulose, cellulose ester, cellulose ether, cellulose acylate, cellulose diacylate, cellulose triacylate, cellulose acetate, cellulose diacetate, cellulose triacetate and a mixture thereof.

14. The pharmaceutical formulation according to claim 13, wherein a content of the water-insoluble polymer is in the range of 0.1 parts by weight to 30 parts by weight, relative to 1 part by weight of the pharmacologically active ingredient.

15. The pharmaceutical formulation according to claim 13, wherein the water-insoluble polymer is at least one selected from polyvinylacetate, ethylcellulose and cellulose acetate.

16. The pharmaceutical formulation according to claim 8, wherein the release-controlling material is at least one selected from a water-insoluble polymer and an enteric polymer.

17. The pharmaceutical formulation according to claim 16, wherein the water-insoluble polymer is at least one selected from polyvinylacetate, ethylcellulose and cellulose acetate, and the enteric polymer is hypromellose phthalate or a methyl acrylate/methacrylic acid copolymer.

18. The pharmaceutical formulation according to claim 8, wherein the hydrophobic compound is at least one selected from a fatty acid or fatty acid ester, a fatty acid alcohol, a wax, an inorganic material, and a mixture thereof.

19. The pharmaceutical formulation according to claim 18, wherein the fatty acid or fatty acid ester is at least one selected from glyceryl palmitostearate, glyceryl stearate, glyceryl behenate, cetyl palmitate, glyceryl monooleate, stearic acid and a mixture thereof; the fatty acid alcohol is at least one selected from cetostearyl alcohol, cetyl alcohol, stearyl alcohol and a mixture thereof; the wax is at least one selected from carnauba wax, beeswax, microcrystalline wax and a mixture thereof; and the inorganic material is at least one selected from talc, precipitated calcium carbonate, calcium hydrogen phosphate, zinc oxide, titanium oxide, kaolin, bentonite, montmorillonite, veegum and a mixture thereof .

20. The pharmaceutical formulation according to claim 18, wherein the content of the hydrophobic compound is in the range of 0.1 parts by weight to 20 parts by weight, relative to 1 part by weight of the pharmacologically active ingredient.

21. The pharmaceutical formulation according to claim 8, wherein the hydrophilic polymer is at least one selected from a saccharide, a cellulose derivative, a gum, a protein, a polyvinyl derivative, a hydrophilic polymethacrylate copolymer, a polyethylene derivative, a carboxyvinyl copolymer and a mixture thereof.

22. The pharmaceutical formulation according to claim 21, wherein the saccharide is at least one selected from dextrin, polydextrin, dextran, pectin and a pectin derivative, alginate, polygalacturonic acid, xylan, arabinoxylan, arabinogalactan, starch, hydroxypropyl starch, amylose, amylopectin and a mixture thereof; the cellulose derivative is at least one selected from hydroxypropylmethylcellulose, hydroxypropylcellulose, hydroxymethylcellulose,
hydroxyethylcellulose, methylcellulose, sodium carboxymethylcellulose, hydroxypropylmethylcellulose acetate succinate, hydroxyethylmethylcellulose and a mixture thereof; the gum is at least one selected from guar gum, locust bean gum, tragacanth, carrageenan, gum acacia, gum arabic, gellan gum, xanthan gum and a mixture thereof; the protein is at least one selected from gelatin, casein, zein and a mixture thereof; the polyvinyl derivative is at least one selected from polyvinyl alcohol, polyvinyl pyrrolidone, polyvinylacetal diethylaminoacetate and a mixture thereof; the hydrophilic polymethacrylate copolymer is at least one selected from a poly(butyl methacrylate, (2-dimethylaminoethyl) methacrylate, methyl methacrylate) copolymer, a poly(methacrylic acid, methyl methacrylate) copolymer, a poly(methacrylic acid, ethyl acrylate) copolymer and a mixture thereof; the polyethylene derivative is at least one selected from polyethylene glycol, polyethylene oxide and a mixture thereof; and the carboxyvinyl polymer is carbomer.

23. The pharmaceutical formulation according to claim 21, wherein the content of the hydrophilic polymer is in the range of 0.05 parts by weight to 30 parts by weight, relative to 1 part by weight of the pharmacologically active ingredient.

24. The pharmaceutical formulation according to any one of claims 1 to 3, wherein the pharmaceutical formulation is an uncoated tablet, a film-coated tablet, a multi-layered tablet, a press-coated tablet, or a capsule.

25. The pharmaceutical formulation according to claim 24, wherein the uncoated tablet is a tablet obtained by compression of granules containing an angiotensin-II-receptor blocker and a release-controlling material.

26. The pharmaceutical formulation according to claim 24, wherein the film-coated tablet is a tablet in which a tablet containing an angiotensin-II-receptor blocker is coated with a film-coated layer containing a release-controlling material.

27. The pharmaceutical formulation according to claim 24, wherein the multi-layered tablet is a tablet having a layered structure of a layer containing an angiotensin-II-receptor blocker and a release-controlling material and a layer containing a release-controlling material.

28. The pharmaceutical formulation according to claim 24, wherein the press-coated tablet is a tablet including an inner core tablet containing an angiotensin-II-receptor blocker and a release-controlling material and an outer layer containing a release-controlling material enclosing the outer surface of the inner core tablet.

29. The pharmaceutical formulation according to claim 24, wherein the press-coated tablet is a tablet including a coating layer enclosing the outer surface of an angiotensin-II-receptor blocker-containing tablet and containing a release-controlling material, and an outer layer enclosing the outer surface of the coating layer and containing a release-controlling material.

30. The pharmaceutical formulation according to claim 24, wherein the press-coated tablet is an osmotic press-coated tablet.

31. The pharmaceutical formulation according to claim 24, wherein the capsule is of a form where at least one selected from a particle, a granule, a pellet and a tablet is filled in a capsule.

32. The pharmaceutical formulation according to any one of claims 1 to 3, wherein the formulation further comprises a coating layer on the outside thereof.

33. The pharmaceutical formulation according to any one of claims 1 to 3, wherein the formulation contains an osmo-regulator and is coated by a semi-permeable membrane coating base.

34. The pharmaceutical formulation according to claim 33, wherein the osmo-regulator is at least one selected from the group consisting of magnesium sulfate, magnesium chloride, sodium chloride, lithium chloride, potassium sulfate, sodium sulfate, lithium sulfate and a mixture thereof.

35. The pharmaceutical formulation according to claim 33, wherein the semi-permeable membrane coating base is at least one selected from the group consisting of polyvinyl acetate, a polymethacrylate copolymer, a poly(ethyl acrylate, methyl methacrylate) copolymer, a poly(ethyl acrylate, methyl methacrylate, trimethylaminoethyl methacrylate chloride) copolymer, ethylcellulose, cellulose ester, cellulose ether, cellulose acylate, cellulose diacylate, cellulose triacylate, cellulose acetate, cellulose diacetate, cellulose triacetate and a mixture thereof.

36. The pharmaceutical formulation according to any one of claims 1 to 3, wherein the formulation is for evening administration.

37. The pharmaceutical formulation according to any one of claims 1 to 3, wherein the pharmaceutical formulation is for administration in combination with a drug which is metabolized by cytochrome.

38. The pharmaceutical formulation according to claim 37, wherein the drug metabolized by cytochrome is at least one selected from a lipid inhibitor which is at least one selected from simvastatin, and atorvastatin; an antihypertensive agent which is at least one selected from amlodipine, felodipine, carvedilol, and aliskiren; an antihistamine agent which is at least one selected from loratadine, and azelastine; an analgesic agent which is at least one selected from acetaminophen, and tramadol; an antidepressant which is at least one selected from amitriptyline, and imipramine; an anticoagulant which is warfarin; an anti-emetic agent which is at least one selected from chlorpromazine, and granisetron; an anticonvulsant which is carbamazepine; an anti-acne agent which is isotretinoin; an antipsychotic agent which is risperidone; an antidepressant which is at least one selected from propyne, and venlafaxine; an HIV-antiviral agent which is fosamprenavir; an antifungal agent which is itraconazole; a thiazolidinedione antidiabetic agent which is pioglitazone; an anti-obesity agent which is sibutramine; an anti-erectile dysfunction agent which is sildenafil; and an anti-incontinence agent which is tolterodine.

39. A pharmaceutical formulation comprising a prior-release compartment containing a hepatitis-preventing and inhibiting agent as a pharmacologically active ingredient, and a delayed-release compartment containing an angiotensin-II-receptor blocker as a pharmacologically active ingredient.

40. The pharmaceutical formulation according to claim 39, wherein the pharmacologically active ingredient contained in the delayed-release compartment is released 1 to 4 hours after the release of the pharmacologically active ingredient contained in the prior-release compartment is initiated.

41. The pharmaceutical formulation according to claim 39, wherein the pharmacologically active ingredient contained in the prior-release compartment is released at a level of more than 85% by weight within one hour after initiation of release thereof.

42. The pharmaceutical formulation according to claim 39, wherein less than 40% by weight of the pharmacologically active ingredient contained in the delayed-release compartment is released within 4 hours after the release of the pharmacologically active ingredient contained in the prior-release compartment is initiated.

43. The pharmaceutical formulation according to claim 39, wherein the content of the pharmacologically active ingredient of the prior-release compartment in the formulation is in the range of 0.01 µg to 1 g.

44. The pharmaceutical formulation according to claim 39, wherein the content of the pharmacologically active ingredient of the delayed-release compartment in the formulation is in the range of 5 to 600 mg.

45. The pharmaceutical formulation according to any one of claims 39 to 44, wherein the delayed-release compartment further comprises a hepatitis-preventing and inhibiting agent as a pharmacologically active ingredient.

46. The pharmaceutical formulation according to claim 45, wherein the hepatitis-preventing and inhibiting agent is at least one selected from β-carotene, riboflavin, riboflavin tetrabutyrate, riboflavin rocoat, riboflavin phosphate sodium, ascorbic acid, ascorbyl palmitate, calcium ascorbate, nicotinamide ascorbate, sodium ascorbate, dehydroascorbic acid, cholecalciferol, cholecalciferolic acid, ergocalciferol, tocopherol, tocopherol acetate, tocopherol calcium, tocopherol calcium succinate, tocopherol succinate, cysteine, cysteine hydrochloride, cysteine malate, methyl cysteine, carboxymethyl cysteine, methyl cysteine hydrochloride, N-acetyl-L-cysteine, L-glutathione, glutathione disulfide, reduced glutathione, L-glutamine, glutamine hydrochloride, L-glutaminate-L-lysinate, L-glutamine, N(2)-L-alanine-L-glutamine, α-lipoic acid, selenized yeast, selenium, selenium disulfide, sodium selenate, sodium selenite, silymarin, ginko biloba extract, biphenyldimethyldicarboxylate, ursodeoxycholic acid, chenocholic acid, butylated hydroxyanisole, butylated hydroxytoluene, guaiacol, erdosteine, resveratrol, pyridoxamine hydrochloride, pyridoxine hydrochloride, pyridoxal phosphate, agaro-oligosaccharide, fraction flavonoid purifiee micronise, melatonin, ubidecarenone, L-ornithine, ornithine aspartate, ornithine hydrochloride, L-arginine, arginine hydrochloride, arginine sodium, L-arginine monoglutamate, protoporphyrin disodium, haematoporphyrin, haematoporphyrin hydrochloride, Coriolus versicolor polysaccharide, cicloxilic acid, citiolone, urazamide, azintamide, hymecromone, and α-naphthyl acetic acid.

47. The pharmaceutical formulation according to any one of claims 39 to 44, wherein the angiotensin-II-receptor blocker is at least one selected from losartan, valsartan, telmisartan, irbesartan, candesartan, olmesartan, eprosartan, isomers thereof, pharmaceutically acceptable salts thereof, and prodrugs thereof.

48. The pharmaceutical formulation according to any one of claims 39 to 44, wherein the delayed-release compartment further comprises at least one release-controlling material selected from an enteric polymer, a water-insoluble polymer, a hydrophobic compound, a hydrophilic polymer and a mixture thereof, in addition to pharmacologically active ingredients.

49. The pharmaceutical formulation according to claim 48, wherein the content of the release-controlling material is in the range of 0.05 to 100 parts by weight, relative to 1 part by weight of the pharmacologically active ingredient.

50. The pharmaceutical formulation according to claim 48, wherein the enteric polymer is at least one selected from the group consisting of an enteric cellulose derivative, an enteric acrylic acid copolymer, an enteric polymethacrylate copolymer, an enteric maleic acid copolymer, an enteric polyvinyl derivative, and a mixture thereof.

51. The pharmaceutical formulation according to claim 50, wherein the enteric cellulose derivative is at least one selected from hydroxypropylmethylcellulose acetate succinate, hydroxypropylmethylcellulose phthalate, hydroxymethylethylcellulose phthalate, cellulose acetate phthalate, cellulose acetate succinate, cellulose acetate maleate, cellulose benzoate phthalate, cellulose propionate phthalate, methylcellulose phthalate, carboxymethylethylcellulose, ethylhydroxyethylcellulose phthalate, methylhydroxyethylcellulose and a mixture thereof; the enteric acrylic acid copolymer is at least one selected from a styrene/acrylic acid copolymer, a methyl acrylate/acrylic acid copolymer, a methyl acrylate/methacrylic acid copolymer, a butyl acrylate/styrene/acrylic acid copolymer, a methyl acrylate/methacrylic acid/octyl acrylate copolymer and a mixture thereof; the enteric polymethacrylate copolymer is at least one selected from a poly(methacrylic acid/methyl methacrylate copolymer), a poly(methacrylic acid/ethyl acrylate) copolymer and a mixture thereof; the enteric maleic acid copolymer is at least one selected from a vinyl acetate/maleic anhydride copolymer, a styrene/maleic anhydride copolymer, a styrene/maleic monoester copolymer, a vinyl methyl ether/maleic anhydride copolymer, an ethylene/maleic anhydride copolymer, a vinyl butyl ether/maleic anhydride copolymer, an acrylonitrile/methyl acrylate/maleic anhydride copolymer, a butyl acrylate/styrene/maleic anhydride copolymer and a mixture thereof; and the enteric polyvinyl derivative is at least one selected from polyvinylalcohol phthalate, polyvinylacetate phthalate, polyvinylbutyrate phthalate, polyvinylacetacetal phthalate and a mixture thereof.

52. The pharmaceutical formulation according to claim 50, wherein the content of the enteric polymer is in the range of 0.1 parts by weight to 20 parts by weight, relative to 1 part by weight of the pharmacologically active ingredient.

53. The pharmaceutical formulation according to claim 48, wherein the water-insoluble polymer is at least one selected from the group consisting of polyvinyl acetate, a water-insoluble polymethacrylate copolymer, ethylcellulose, cellulose ester, cellulose ether, cellulose acylate, cellulose diacylate, cellulose triacylate, cellulose acetate, cellulose diacetate, cellulose triacetate and a mixture thereof.

54. The pharmaceutical formulation according to claim 53, wherein the content of the water-insoluble polymer is in the range of 0.1 parts by weight to 30 parts by weight, relative to 1 part by weight of the pharmacologically active ingredient.

55. The pharmaceutical formulation according to claim 48, wherein the water-insoluble polymer is at least one selected from polyvinylacetate, ethylcellulose and cellulose acetate.

56. The pharmaceutical formulation according to claim 48, wherein the release-controlling material is at least one selected from a water-insoluble polymer and an enteric polymer.

57. The pharmaceutical formulation according to claim 56, wherein the water-insoluble polymer is at least one selected from polyvinylacetate, ethylcellulose and cellulose acetate, and the enteric polymer is at least one selected from hydroxypropylmethylcellulose phthalate and a methyl acrylate/methacrylic acid copolymer.

58. The pharmaceutical formulation according to claim 48, wherein the hydrophobic compound is at least one selected from a fatty acid or fatty acid ester, a fatty acid alcohol, a wax, an inorganic material, and a mixture thereof.

59. The pharmaceutical formulation according to claim 58, wherein the fatty acid or fatty acid ester is at least one selected from glyceryl palmitostearate, glyceryl stearate, glyceryl behenate, cetyl palmitate, glyceryl monooleate, stearic acid and a mixture thereof; the fatty acid alcohol is at least one selected from cetostearyl alcohol, cetyl alcohol, stearyl alcohol and a mixture thereof; the wax is at least one selected from carnauba wax, beeswax, microcrystalline wax and a mixture thereof; and the inorganic material is at least one selected from talc, precipitated calcium carbonate, calcium hydrogen phosphate, zinc oxide, titanium oxide, kaolin, bentonite, montmorillonite, veegum and a mixture thereof.

60. The pharmaceutical formulation according to claim 58, wherein the content of the hydrophobic compound is in the range of 0.1 parts by weight to 20 parts by weight, relative to 1 part by weight of the pharmacologically active ingredient.

61. The pharmaceutical formulation according to claim 48, wherein the hydrophilic polymer is at least one selected from a saccharide, a cellulose derivative, a gum, a protein, a polyvinyl derivative, a hydrophilic polymethacrylate copolymer, a polyethylene derivative, a carboxyvinyl copolymer and a mixture thereof.

62. The pharmaceutical formulation according to claim 61, wherein the saccharide is at least one selected from dextrin, polydextrin, dextran, pectin and a pectin derivative, alginate, polygalacturonic acid, xylan, arabinoxylan, arabinogalactan, starch, hydroxypropyl starch, amylose, amylopectin and a mixture thereof; the cellulose derivative is at least one selected from hydroxypropylmethylcellulose, hydroxypropylcellulose, hydroxymethylcellulose, hydroxyethylcellulose, methylcellulose, sodium carboxymethylcellulose, hydroxypropylmethylcellulose acetate succinate, hydroxyethylmethylcellulose and a mixture thereof; the gum is at least one selected from guar gum, locust bean gum, tragacanth, carrageenan, gum acacia, gum arabic, gellan gum, xanthan gum and a mixture thereof; the protein is at least one selected from gelatin, casein, zein and a mixture thereof; the polyvinyl derivative is at least one selected from polyvinyl alcohol, polyvinyl pyrrolidone, polyvinylacetal diethylaminoacetate and a mixture thereof; the hydrophilic polymethacrylate copolymer is at least one selected from a poly(butyl methacrylate, (2-dimethylaminoethyl) methacrylate, methyl methacrylate) copolymer, a poly(methacrylic acid, methyl methacrylate) copolymer, a poly(methacrylic acid, ethyl acrylate) copolymer and a mixture thereof; the polyethylene derivative is at least one selected from polyethylene glycol, polyethylene oxide and a mixture thereof; and the carboxyvinyl polymer is carbomer.

63. The pharmaceutical formulation according to claim 61, wherein the content of the hydrophilic polymer is in the range of 0.05 parts by weight to 30 parts by weight, relative to 1 part by weight of the pharmacologically active ingredient.

64. The pharmaceutical formulation according to any one of claims 39 to 44, wherein the pharmaceutical formulation is in the form of a two-phase matrix tablet which is obtained by uniformly mixing a delayed-release compartment and a prior-release compartment, followed by compression.

65. The pharmaceutical formulation according to any one of claims 39 to 44, wherein the pharmaceutical formulation is in the form of a film-coated tablet including a tablet consisting of a delayed-release compartment and a film-coated layer consisting of a prior-release compartment enclosing the exterior of the tablet.

66. The pharmaceutical formulation according to any one of claims 39 to 44, wherein the pharmaceutical formulation is in the form of a multi-layered tablet having a multi-layered structure of the delayed-release compartment and the prior-release compartment.

67. The pharmaceutical formulation according to any one of claims 39 to 44, wherein the pharmaceutical formulation is in the form of a press-coated tablet including an inner core tablet consisting of a delayed-release compartment and an outer layer consisting of a prior-release compartment enclosing the outer surface of the inner core tablet.

68. The pharmaceutical formulation according to claim 67, wherein the press-coated tablet is an osmotic press-coated tablet.

69. The pharmaceutical formulation according to any one of claims 39 to 44, wherein the pharmaceutical formulation is in the form of a capsule including a particle, granule, pellet, or tablet consisting of a delayed-release compartment and a particle, granule, pellet, or tablet consisting of a prior-release compartment.

70. The pharmaceutical formulation according to any one of claims 39 to 44, further comprising a coating layer on the outside of the delayed-release compartment and/or the prior-release compartment.

71. The pharmaceutical formulation according to any one of claims 39 to 44, wherein the delayed-release compartment is a compartment which contains an osmo-regulator and is coated by a semi-permeable membrane coating base.

72. The pharmaceutical formulation according to claim 71, wherein the osmo-regulator is at least one selected from the group consisting of magnesium sulfate, magnesium chloride, sodium chloride, lithium chloride, potassium sulfate, sodium sulfate, lithium sulfate and a mixture thereof.

73. The pharmaceutical formulation according to claim 71, wherein the semi-permeable membrane coating base is at least one selected from the group consisting of polyvinyl acetate, a polymethacrylate copolymer, a poly(ethyl acrylate, methyl methacrylate) copolymer, a poly(ethyl acrylate, methyl methacrylate, trimethylaminoethyl methacrylate chloride) copolymer, ethylcellulose, cellulose ester, cellulose ether, cellulose acylate, cellulose diacylate, cellulose triacylate, cellulose acetate, cellulose diacetate, cellulose triacetate and a mixture thereof.

74. The pharmaceutical formulation according to any one of claims 39 to 44, wherein the formulation is in the form of a coated tablet further including a coating layer on the outside thereof.

75. The pharmaceutical formulation according to any one of claims 39 to 44, wherein the formulation is in the form of a kit including a delayed-release compartment and a prior-release compartment.

76. The pharmaceutical formulation according to any one of claims 39 to 44, wherein the formulation is for evening administration.

77. The pharmaceutical formulation according to any one of claims 39 to 44, wherein the pharmaceutical formulation is for administration in combination with a drug which is metabolized by cytochrome.

78. The pharmaceutical formulation according to claim 77, wherein the drug metabolized by cytochrome is at least one selected from a lipid inhibitor which is at least one selected from simvastatin, and atorvastatin; an antihypertensive agent which is at least one selected from amlodipine, felodipine, carvedilol, and aliskiren; an antihistamine agent which is at least one selected from loratadine, and azelastine; an analgesic agent which is at least one selected from acetaminophen, and tramadol; an antidepressant which is at least one selected from amitriptyline, and imipramine; an anticoagulant which is warfarin; an anti-emetic agent which is at least one selected from chlorpromazine, and granisetron; an anticonvulsant which is carbamazepine; an anti-acne agent which is isotretinoin; an antipsychotic agent which is risperidone; an antidepressant which is at least one selected from propyne, and venlafaxine; an HIV-antiviral agent which is fosamprenavir; an antifungal agent which is itraconazole; a thiazolidinedione antidiabetic agent which is pioglitazone; an anti-obesity agent which is sibutramine; an anti-erectile dysfunction agent which is sildenafil; and an anti-incontinence agent which is tolterodine.
